# EUROPEAN PATENT SPECIFICATION

(11) **EP 2 213 325 B1**
(45) Date of publication and mention of the grant of the patent: **23.11.2016**
(21) Application number: 08842561.6
(22) Date of filing: 24.10.2008
(51) Int. Cl.: A61M 25/00

(54) **CATHETER**
KATHETER
CATHÉTER

(30) Priority: 26.10.2007 JP 2007279688
(43) Date of publication of application: 04.08.2010
(73) Proprietor: Terumo Kabushiki Kaisha, Tokyo 151-0072 (JP)
(72) Inventor: TAKESHITA, Satoshi, Tokyo 165-0031 (JP); TAKAGI, Ayumu, Shizuoka 409-3853 (JP); ISE, Hiroyoshi, Shizuoka 409-3853 (JP); FUKUOKA, Tetsuya, Shizuoka 409-3853 (JP)
(74) Representative: Prüfer & Partner mbB Patentanwälte · Rechtsanwälte
(86) International application number: PCT/JP2008/069369
(87) International publication number: WO 2009/054509

(56) References cited:
- EP-A1- 1 698 369
- EP-A1- 1 712 247
- WO-A1-2005/056100
- JP-A- 2004 357 805
- US-A- 5 725 513
- US-A- 6 045 734
- US-B1- 6 740 073
- US-B1- 6 858 024

## Description

### Technical Field

The present invention relates to a catheter used in the state of being inserted in a heart or its peripheral tissue, particularly, into a left or right coronary artery (hereinafter, it may also be referred to simply as "coronary artery").

### Background Art

As guiding catheters, in general, those which are comparatively large in inside diameter have been demanded, in order to cope with a diversity of lesions. This is because a medical device being large in outside diameter, such as Rotablator, is needed to treat a severe lesion having undergone calcification.

On the contrary, in the case of a slight lesion that is located near periphery, in order to lessen damages to blood vessel walls and suppress generation of thrombus, treatment may be conducted using a balloon, a stent and a guide wire smaller in outside diameter together with a guiding catheter having a comparatively small outside diameter, which is advantageous in view of less side effects. In addition, a smaller-bore catheter is more flexible at the tip (distal end) and, therefore, less liable to damage blood vessel walls, as compared with a larger-bore catheter.

In recent years, as guiding catheters for PTCA, those having an outside diameter of 1.67 to 2.67 mm (5 to 8 Fr) (in Japan, mostly 6 or 7 Fir; 1 Fr corresponds to about 0.33 mm) are commonly used (refer to, for example, Patent Document 1). In introducing such a catheter into a living body, the sheath to be used to introduce the catheter having a large outside diameter is naturally large in outside diameter, and the puncture formed by the catheter introducer sheath is also large accordingly. Therefore, after the treatment using such a catheter, it takes much time for hemostasis at the puncture after the sheath is pulled out, and the resting time for this purpose will be long.

Besides, when the catheter having a large outside diameter as above is gradually pushed into a blood vessel, the blood vessel wall may be damaged upon passage of the catheter through the blood vessel. Further, in the case of a catheter of 6 Fr (outside diameter: 2.0 mm) or more in size, the large outside diameter would make it impossible to insert the catheter into the vicinity of a lesion when the lesion is located at the periphery of a coronary artery. Consequently, the medical device (balloon, stent, or the like) guided through the lumen of the guiding catheter is passed inside the coronary artery in a naked state. In this instance, if a trace of treatment such as indwelling of a stent is already present on the operator's side of the lesion (the proximal side of the lesion in the coronary artery), the guide wire or the tip of the balloon being slender at the tip may be caught on the indwelling stent and, hence, cannot reach the lesion.

As in the case of a catheter assembly (combined catheter) composed of an outer catheter and an inner catheter inserted in the outer catheter, there is also a method in which an inner catheter smaller in outside diameter than an outer catheter whose tip is to be engaged with (hooked on) an entrance of a target blood vessel is inserted in the outer catheter and the inner catheter is used for treatment of the lesion located at the periphery of the target blood vessel. However, this method is the same as the above-mentioned in that a sheath with a large outside diameter is used for insertion of the outer catheter, and, accordingly, the puncture formed for introducing the catheter is large.

When it is attempted to treat a lesion by using alone a catheter of 5 Fr (outside diameter: 1.7 mm) or more in size, it is necessary for the catheter to be provided at its tip with such a shape that the tip can be engaged with the entrance to a target blood vessel. With the 1.67 mm (5 Fr) catheter, however, there is a risk of closing the inside diameter of the target blood vessel. In addition, since the 1.67 mm (5 Fr) catheter is advanced in the blood vessel while keeping the engageable shape at its distal end, the distal portion (in the engageable shape) of the catheter may be caught on a curved portion of the target blood vessel, possibly damaging the blood vessel wall considerably.

EP 1 698 369 A1 discloses a catheter assemble comprising an outer catheter and an inner catheter wherein the outer catheter is configured to provide passage for the inner catheter.

US 6 740 073 B1 discloses a guiding catheter for intraluminal procedures which is by its dimensional layout suited to be seated in the ostium of a desired coronary artery. It is further configured and adapter to provide coronary artery access for diagnostic devices such as a balloon dilation catheter or a stent delivery catheter.

US 6 858 024 B1 describes a guiding catheter with an outer tubular member overlaying the braid and inner tubular member. The outer member of the catheter is then contoured providing improved back-out support for the guide catheter during coronary treatment. Similarly, EP 1 712 247 A1 describes a catheter comprising a catheter main body with a curved part at the distal end of the catheter which is configured and adapted to be engaged with a coronary ostium and to provide back-up force to not disengage from the coronary ostium when the PTCA catheters or the like advances to the depth of the coronary here.

US 6 045 734 discloses a catheter of various small dimensions for the use in neonatal care comprising a mandrel, an inner sleeve, a middle sleeve and an outer sleeve, however missing a curved part at the distal end of the catheter.

US 5 725 513 A discloses a catheter constituted of two layers wherein in the outer layer a braided sleeve of metal wire is incorporated. The catheter further comprises a soft-tip member at the end of a curved part at the distal side of the catheter.

A guiding catheter having a small outside diameter which, when used alone, is capable of engaging with the entrance of a target blood vessel, capable of advancing into the blood vessel after the engagement and capable of feeding forward a medical device therethrough and which can be deformed according to the shape of the target blood vessel up to the vicinity of a lesion in the blood vessel, has not yet been known.

Patent Document 1: Japanese Patent Laid-open No. 2006-288670

### Disclosure of Invention

It is an object of the present invention to provide a catheter which can be assuredly inserted to a target site in a blood vessel through a simple operation and in a safe and assured manner.

In order to attain the above object, according to the present invention, there is provided a guiding catheter as defined by claim 1.

This ensures that the catheter can be assuredly inserted into a target site in a blood vessel through a simple operation and in a safe and assured manner.

In addition, in the catheter according to the present invention, preferably, the minimum thickness of the outer layer is the distance from an outermost periphery of the reinforcing material layer to an outside surface of the outer layer.

This ensures that the curved part can be assuredly engaged with a branched portion of a blood vessel, and, when the catheter is pushed forward starting from the engaged state, its curved part is deformed according to the curved shape of the blood vessel, so that the operation (pushing-in operation) can be carried out easily and securely. Thus, the catheter can be inserted into a target site through a simple operation and in a safe and assured manner.

Besides, in the catheter according to the invention, preferably, let the average minimum thickness of the outer layer be t and let the outside diameter ø of the catheter body be d, then d/t is in the range of from 50 to 250.

This ensures that the curved part can be assuredly engaged with a branched portion of a blood vessel, and, when the catheter is pushed forward starting from the engaged state, its curved part is deformed according to the curved shape of the blood vessel, so that the operation (pushing-in operation) can be carried out easily and securely. Thus, the catheter can be inserted into a target site through a simple operation and in a safe and assured manner.

In addition, in the catheter according to the invention, preferably, let the flexural rigidity of a proximal portion of the catheter body be σ₁ [gf] and let the crushing strength of the proximal portion be σ₂ [gf], then the condition of σ₁/σ₂ ≤ 0.04 is satisfied.

This ensures that, for example, when the catheter body is inserted into a coronary artery and is passed through a curved portion in the coronary artery, unwilling deformation (kinking (sharp bending), twisting, crushing or the like) of a proximal-side portion of the catheter body can be prevented from occurring. Therefore, steerability and safety at the time of inserting the catheter can be secured sufficiently.

In the catheter according to the invention the catheter body is so designed that the flexural rigidity of a distal-side portion thereof is lower than the flexural rigidity of a proximal-side portion thereof.

This ensures that the catheter body shows little difference (comparatively small difference) in flexural rigidity between its distal-side portion and its proximal-side portion, so that appropriate rigidity (flexibility) can be obtained over the whole part of the catheter body.

In the catheter according to the invention the flexural rigidity of the distal-side portion of the catheter body is smaller than the flexural rigidity of the proximal-side portion of the catheter body by 49.03 to 196.13•10⁻³ N (5 to 20 gf).

This ensures that the catheter body shows little difference (comparatively small difference) in flexural rigidity between its distal-side portion and its proximal-side portion, so that appropriate rigidity (flexibility) can be obtained over the whole part of the catheter body.

Besides, in the catheter according to the invention, preferably, the outer layer is formed from different two or more materials along a longitudinal direction of the catheter body.

This ensures that the catheter body is gradually varied (lowered) in rigidity, or varied (increased) in flexibility, along the distal direction. As a result, during an operation of inserting the catheter into a blood vessel, the catheter can be inserted into the blood vessel more safely while sufficiently securing pushability and torque transmission performance in the distal side.

In addition, in the catheter according to the invention, preferably, the outside diameter of the catheter body is 1.4 to 1.5 mm.

This ensures that the catheter can be used satisfactorily as a guiding catheter.

Besides, in the catheter according to the invention, preferably, the inside diameter of the catheter body is 1.2 to 1.5 mm.

This enables the catheter to be used satisfactorily as a guiding catheter.

In addition, in the catheter according to the invention, preferably, the outer layer is provided in its surface with a multiplicity of recesses.

This ensures that, when the catheter body is inserted into a blood vessel, the area of contact of the surface of the outer layer of the catheter with the blood vessel wall is comparatively small. This, in turn, ensures that sliding resistance (friction) generated between the blood vessel wall and the surface of the outer layer of the catheter can be reduced, that is, slidability of the catheter is enhanced. Accordingly, the curved part can be assuredly inserted into a target site in the blood vessel.

Besides, in the catheter according to the invention, preferably, the average of maximum depths of the recesses is not more than 10 µm.

This ensures that, even if part of the blood wall vessel has entered into the recess or recesses, the part is securely prevented from making contact with inside surfaces of the recess or recesses, so that the contact area between the blood vessel wall and the outer layer of the catheter can be prevented from increasing.

In addition, in the catheter according to the invention, preferably, the curved part is a part having a shape which is curved only toward the same direction.

This ensures that the curved part can be assuredly engaged with a branched portion of a blood vessel, and can assuredly maintain the engaged state.

Besides, in the catheter according to the invention, preferably, the curved part is so configured that a portion having a curvature increasing along the distal direction and a portion having a curvature decreasing along the distal direction are alternately arranged at least once along the longitudinal direction of the catheter body.

This ensures that the curved part is easily engageable with a branched portion of a blood vessel and that the curved part can be more easily deformed from the engaged state into a rectilinear state.

In addition, in the catheter according to the invention, preferably, at least part of the inner layer is formed from a low-friction material.

This ensures that, at the time of inserting a medical long body such as a guide wire or a balloon catheter into the catheter body, longitudinal movement or rotation of the medical long body relative to the catheter body can be performed under smaller sliding resistance, which contributes to enhancement of steerability.

Besides, in the catheter according to the invention, preferably, the soft tip is formed from the same material as that of the outer layer and is formed as one body with the outer layer.

This enables the soft tip to be configured (formed) easily. In addition, since the boundary part between the soft tip and the catheter body (outer layer) is deformed smoothly, the curved part can be deformed more suitably according to the curved shape of a blood vessel when passing in the blood vessel. As a result, the curved part can be assuredly inserted into a target site in a blood vessel through a simple operation.

In addition, the catheter according to the invention, preferably, is a guiding catheter for introducing a balloon catheter, a stent transport catheter or a vasodilation catheter.

This ensures that the catheter can be assuredly inserted into a target site in a blood vessel through a simple operation and in a safe and assured manner.

Besides, preferably, the average total thickness of the inner layer, the outer layer and the reinforcing material layer is 75 to 85 µm.

This ensures that the catheter can be more assuredly inserted into a target site in a blood vessel through a simple operation and in a safe and assured manner.

The catheter can be passed through a lumen of a tubular medical device having an inside diameter of 2.2 to 3.0 mm when disposed inside the distal-side portion of the blood vessel, especially the coronary artery.

This ensures that the catheter can be more assuredly inserted into a target site in a blood vessel through a simple operation and in a safe and assured manner, so that the procedure by use of the medical device can be carried out assuredly.

Besides, preferably, at least part of an outside surface of the outer layer at a distal portion of the catheter body is provided with a coating which exhibits swellability upon contact with a liquid.

This ensures that, when the distal portion of the catheter body makes contact with a liquid, its outside diameter increases. As a result, the outside surface of the distal portion of the catheter body comes into firm contact with, for example, a coronary artery orifice.

In addition, the catheter according to the present invention can be used for the following method: a method of disposing a treatment catheter in a blood vessel,
including:
inserting step of inserting a distal end of a catheter according to the present invention into the inside of a coronary artery;
advancing step of advancing the distal end of the catheter into a peripheral blood vessel of the coronary artery;
protruding step of protruding the treatment catheter from the distal end of the catheter so as to introduce the treatment catheter into the inside of the coronary artery; and
disposing step of disposing the treatment catheter in the inside of the coronary artery.

This enables assured insertion of the catheter into a target site in a blood vessel through a simple operation and in a safe and assured manner.

Besides, in the disposing method, preferably, the treatment catheter is a balloon catheter, a stent transport catheter or a vasodilation catheter.

This enables assured insertion of the catheter into a target site in a blood vessel through a simple operation and in a safe and assured manner.

In order to attain the above object,
there is provided a method for treating a lesion part on the distal side of a treated part in a blood vessel, including:
serving step of serving a single guiding catheter which has a curved shape at its distal end and is engageable with a branched portion of the blood vessel due to the curved shape;
engaging step of engaging the guiding catheter with the branched portion in the blood vessel;
passing step of passing the distal end of the guiding catheter through the inside of the treated part in the blood vessel located on the distal side of the branched portion of the blood vessel to the distal side;
protruding step of protruding an intravascular device from the distal end of the catheter so as to guide the intravascular device to the distal side of the treated part;-and
applying step of applying the intravascular device to the lesion part on the distal side of the treated part.

This enables assured insertion of the catheter, and hence the intravascular device, to a target site in a blood vessel through a simple operation and in a safe and assured manner.

In addition, in the treating method, preferably, the blood vessel is a coronary artery.

This enables assured insertion of the catheter, and hence the intravascular device, to a target site in a blood vessel through a simple operation and in a safe and assured manner.

Besides, in the treating method, preferably, the branched portion of the blood vessel is an entrance to the coronary artery.

This enables the guiding catheter to be assuredly engaged with the entrance to the coronary artery.

In addition, in the treating method, preferably, the guiding catheter is a catheter according to the present invention.

This enables assured insertion of the catheter, and hence the intravascular device, to a target site in a blood vessel through a simple operation and in a safe and assured manner.

Besides, in the treating method, preferably, the intravascular device is a balloon catheter, a stent transport catheter or a vasodilation catheter.

This enables assured insertion of the catheter, and hence the intravascular device, to a target site in a blood vessel through a simple operation and in a safe and assured manner.

### Brief Description of the Drawings

[FIG. 1]

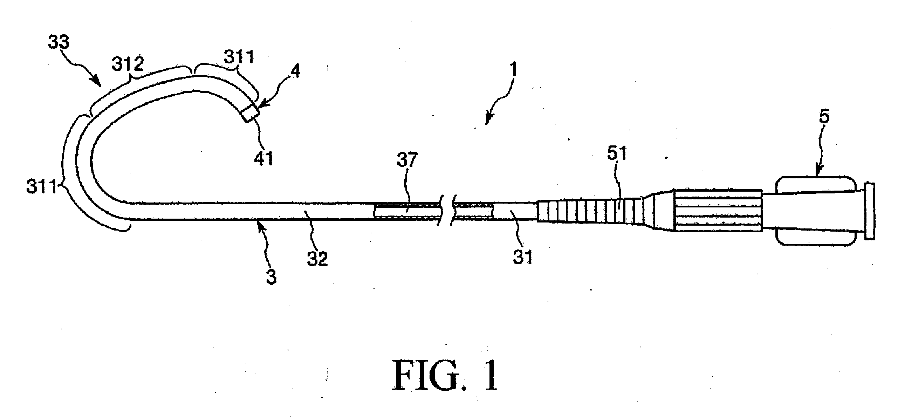

   FIG. 1 is a plan view showing the general shape of a catheter (first embodiment) according to the present invention.
[FIG. 2]

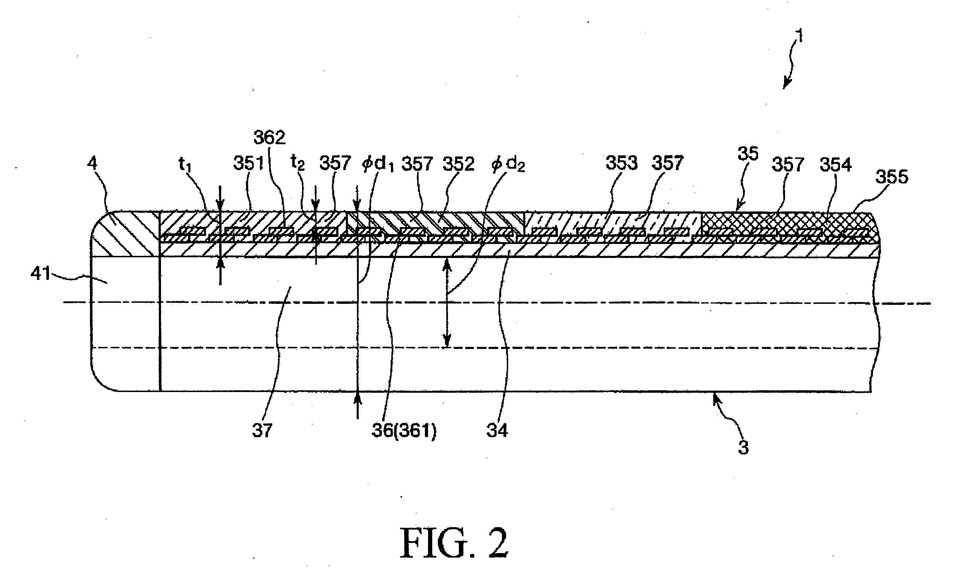

   FIG. 2 is a longitudinal sectional view of a distal portion of the catheter shown in FIG. 1.
[FIG. 3]

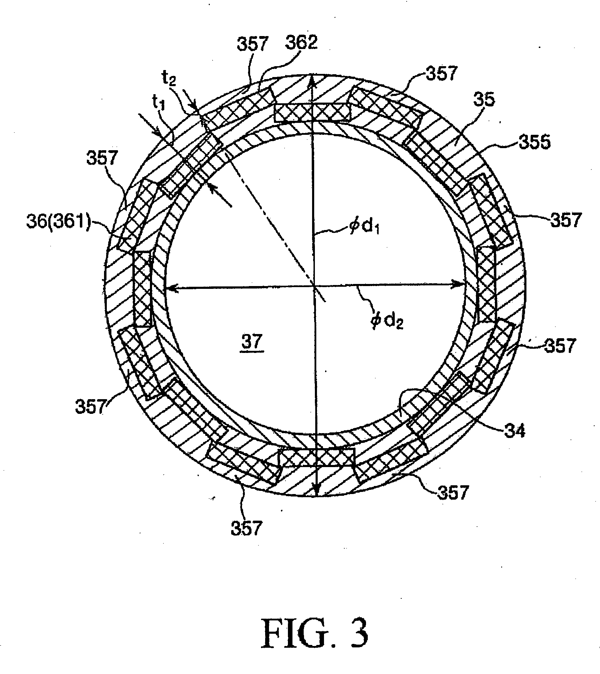

   FIG. 3 is a transverse sectional view of the distal portion of the catheter shown in FIG. 1.
[FIG. 4]

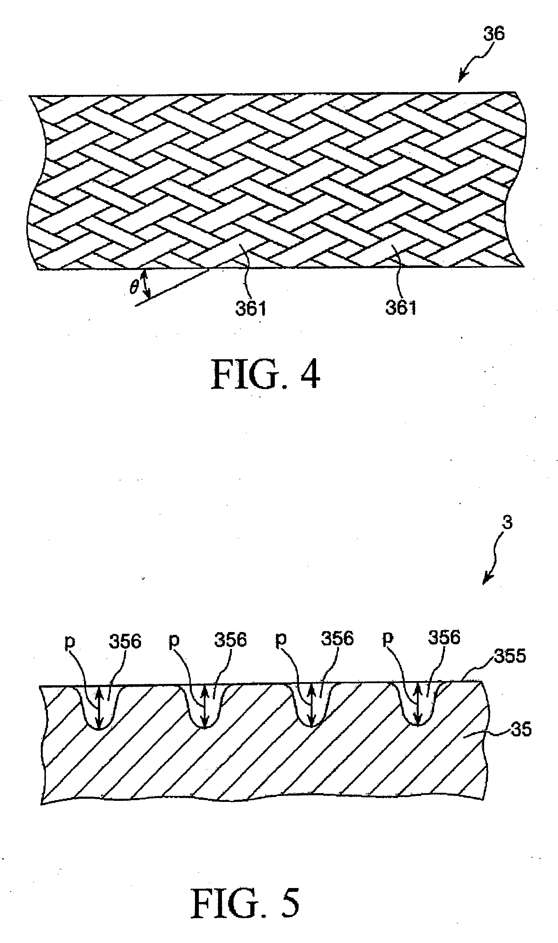

   FIG. 4 is a plan view of a reinforcing material layer in the catheter shown in FIG. 1.
[FIG. 5]
   FIG. 5 is an enlarged longitudinal sectional view of the vicinity of a surface of an outer layer in the catheter shown in FIG. 1.
[FIG. 6]

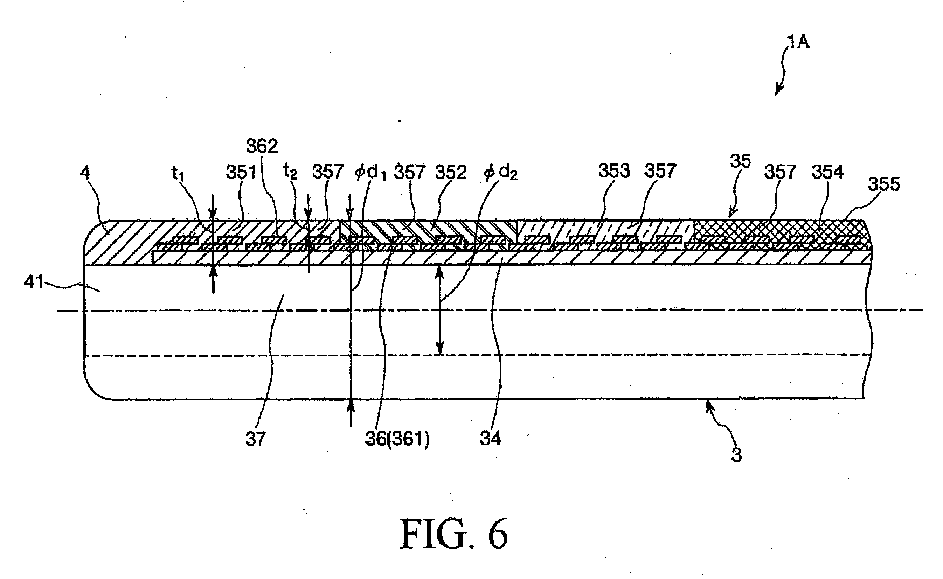

   FIG. 6 is a longitudinal sectional view of a distal portion of a catheter (second embodiment) according to the present invention.
[FIG. 7]

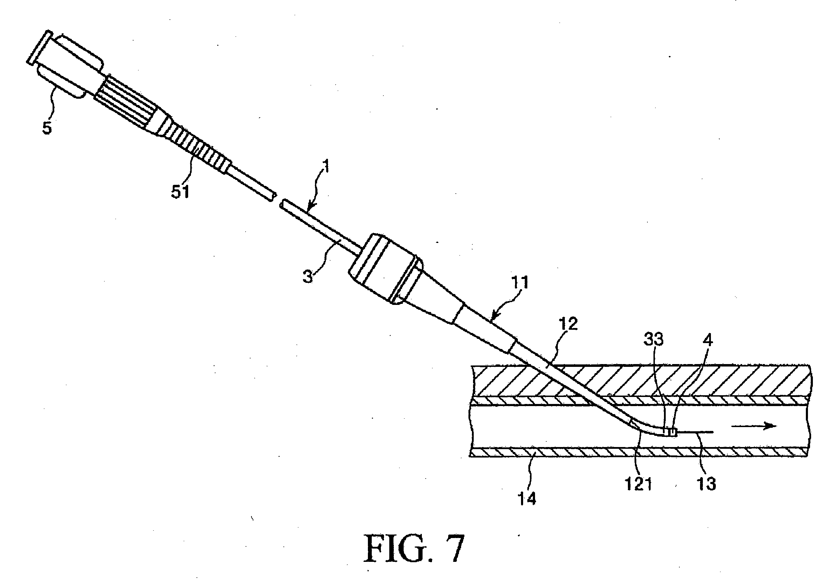

   FIG. 7 illustrates a method of introducing the catheter shown in FIG. 1 into a blood vessel.
[FIG. 8]

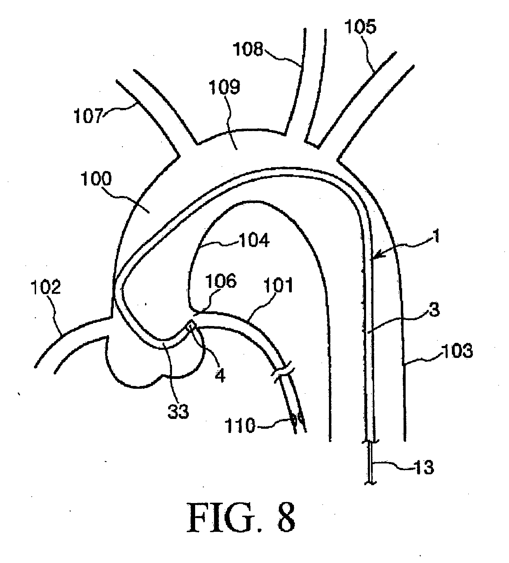

   FIG. 8 illustrates schematically a method of using the catheter shown in FIG. 1.
[FIG. 9]

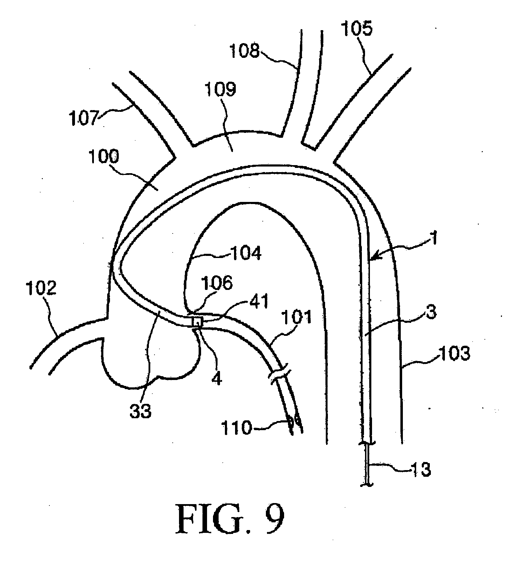

   FIG. 9 illustrates schematically the method of using the catheter shown in FIG. 1.
[FIG. 10]

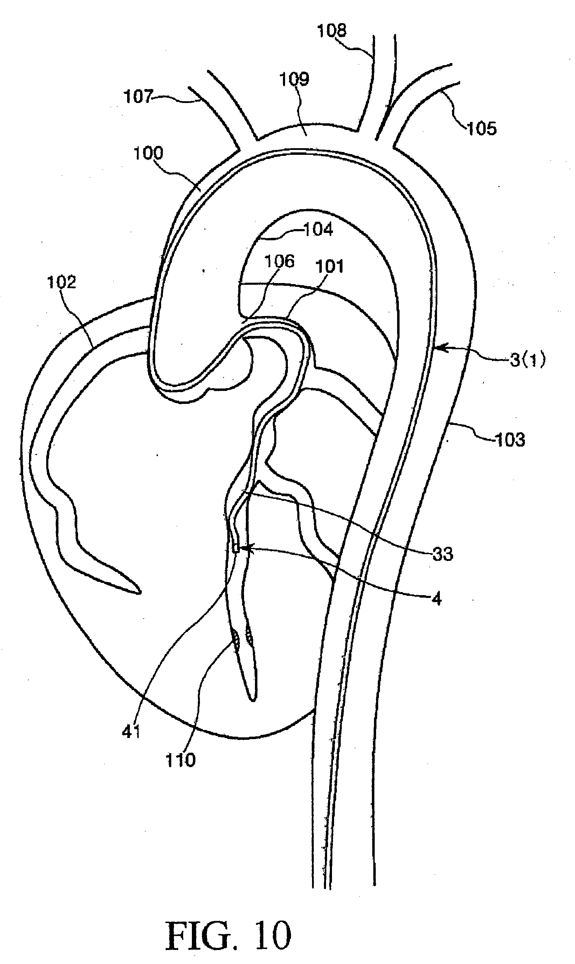

   FIG. 10 illustrates schematically the method of using the catheter shown in FIG. 1.
[FIG. 11]

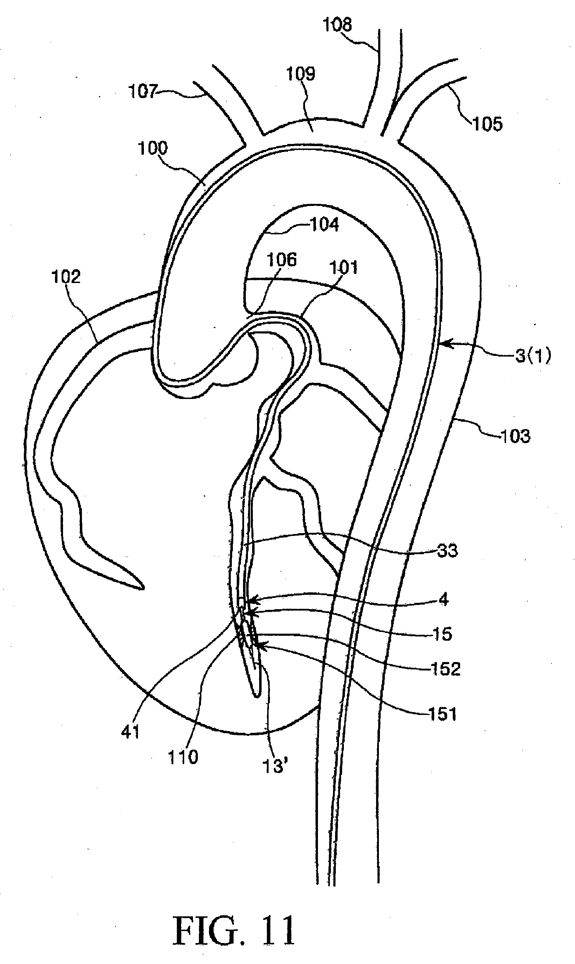

   FIG. 11 illustrates schematically the method of using the catheter shown in FIG. 1.
[FIG. 12]

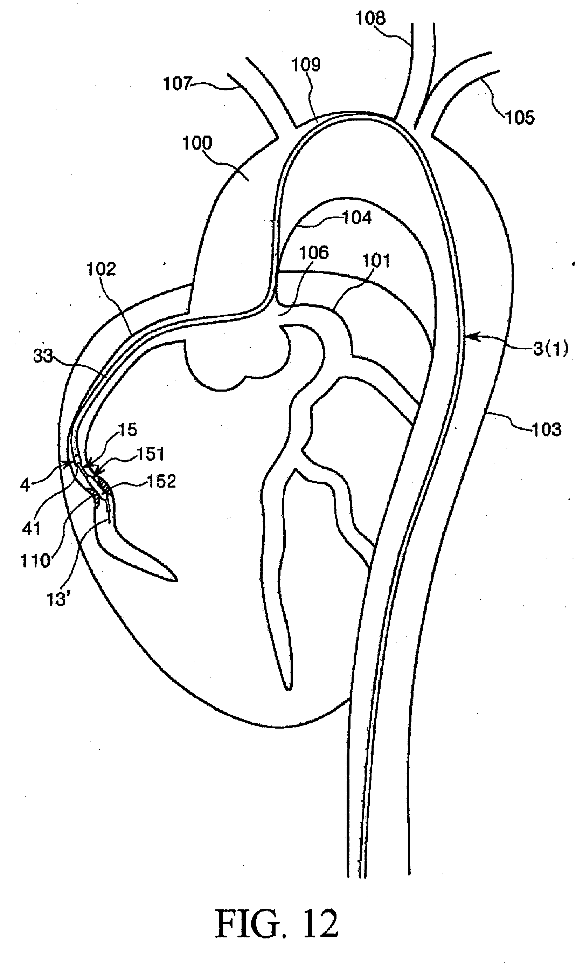

   FIG. 12 illustrates schematically the method of using the catheter shown in FIG. 1.
[FIG. 13]

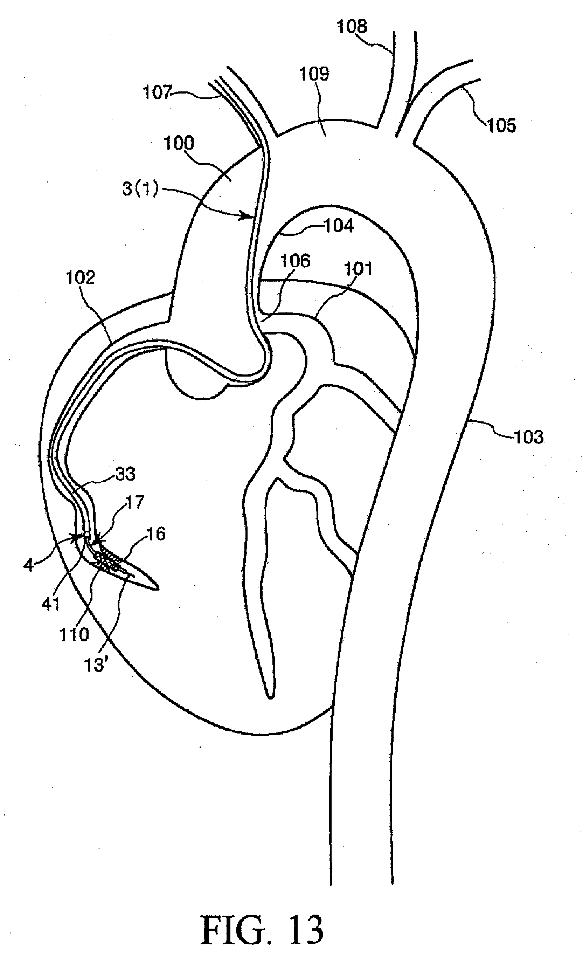

   FIG. 13 illustrates schematically the method of using the catheter shown in FIG. 1.
[FIG. 14]

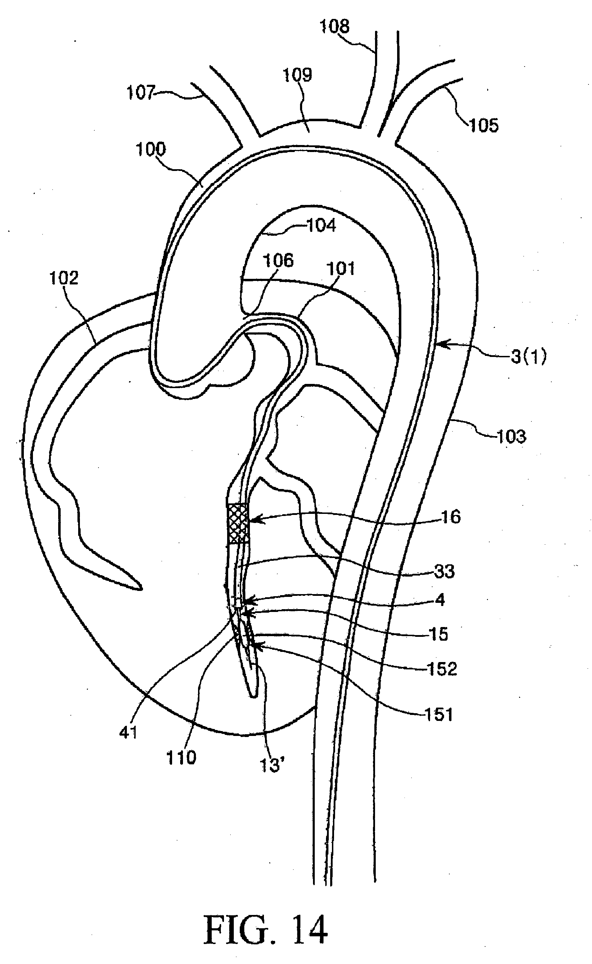

   FIG. 14 illustrates schematically the method of using the catheter shown in FIG. 1.
[FIG. 15]

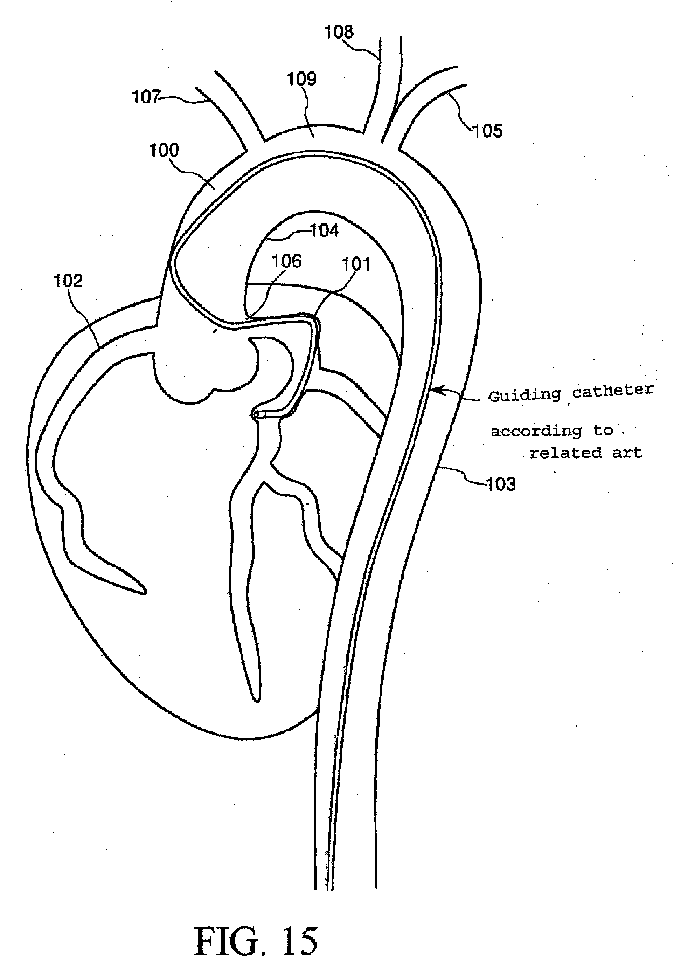

   FIG. 15 is a schematic illustration of a condition where a guiding catheter according to the related art is inserted into a deep part of a coronary artery.
[FIG. 16]

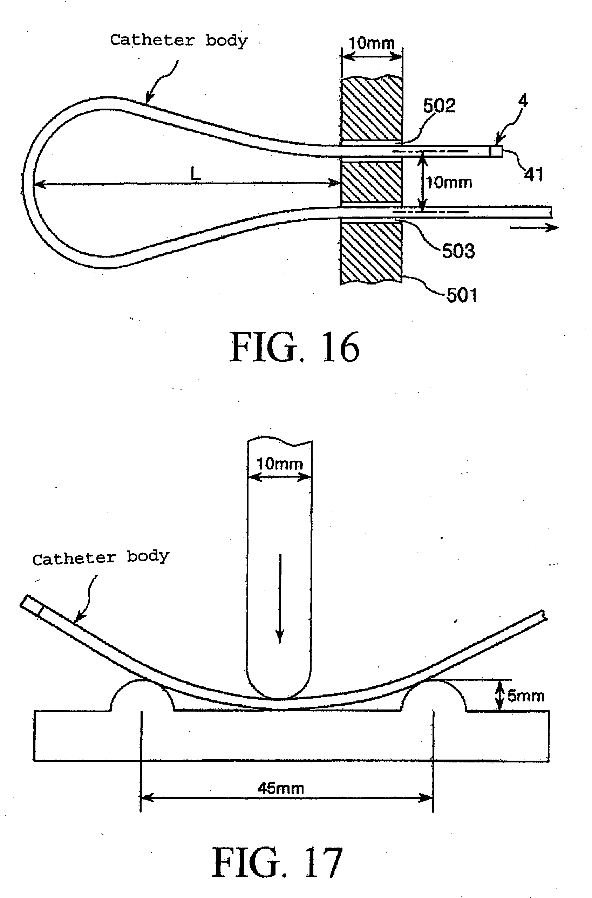

   FIG. 16 illustrates a test method for examining anti-kinking property.
[FIG. 17]
   FIG. 17 illustrates a test method for examining flexural rigidity.
[FIG. 18]

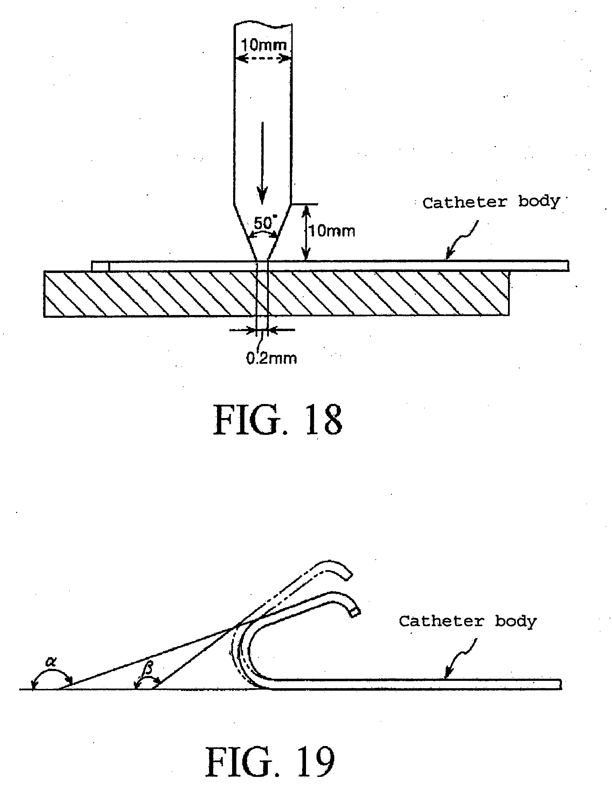

   FIG. 18 illustrates a test method for examining crushing strength.
[FIG. 19]
   FIG. 19 illustrates a test method for examining shape restoration performance of a curved part.
[FIG. 20]

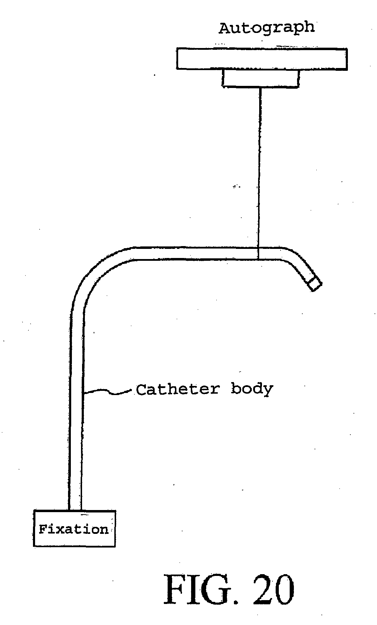

   FIG. 20 illustrates a test method for examining back-up force.
[FIG. 21]

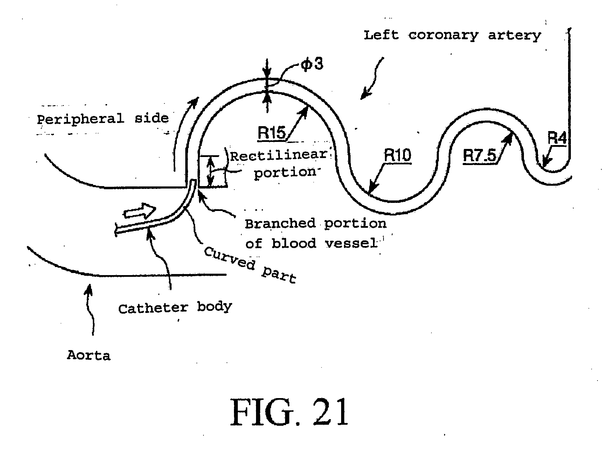

   FIG. 21 illustrates a test method for examining blood vessel trackability of catheter.

### Best Mode for Carrying Out the Invention

Now, the catheter according to the present invention will be described in detail below, based on preferred embodiments shown in the accompanying drawings.

### <First Embodiment>

FIG. 1 is a plan view showing the general shape of a catheter (first embodiment) according to the present invention; FIG. 2 is a longitudinal sectional view of a distal portion of the catheter shown in FIG. 1; FIG. 3 is a transverse sectional view of the distal portion of the catheter shown in FIG. 1; FIG. 4 is a plan view of a reinforcing material layer in the catheter shown in FIG. 1; FIG. 5 is an enlarged longitudinal sectional view of the vicinity of a surface of an outer layer in the catheter shown in FIG. 1; FIG. 7 illustrates a method of introducing the catheter shown in FIG. 1 into a blood vessel; and FIGS. 8 to 14 illustrates schematically a method of using the catheter shown in FIG. 1. Herein, the right side in FIGS. 1 and 2 is taken as "proximal end," and the left side as "distal end," the side near the proximal end is referred to as "proximale," and the side away from the proximal end as "distal" in the following description.

The catheter 1 shown in FIG. 1 is to be used as a guiding catheter for guiding a treatment (remedy) catheter such as a dilation catheter (balloon catheter), a stent transport catheter and a vasodilation catheter into a target site (lesion site) such as a stenosis of a coronary artery (in a blood vessel). Here, the "dilation catheter" is a catheter which, in treatment of a stenosis in a blood vessel, is inserted into the blood vessel, so as to dilate the stenosis by inflating a balloon, for improving the bloodstream on the distal side of the stenosis. Besides, the "stent transport catheter" is a catheter which, in treatment of the stenosis in a blood vessel, is used to transport a stent in a contracted state to the stenosis, to radially expand the stent in the stenosis and to set the expanded stent indwelling in the stenosis, for dilating the stenosis. Further, the "vasodilation catheter" is a catheter for dilating a blood vessel by grinding the inside wall of the blood vessel or making cuts in the blood vessel inside wall, for example, a Rotablator, a cutting balloon, etc.

In general, a distal portion of a guiding catheter is not inserted to the depth side (the vicinity of a stenosis) relative to the entrance (coronary artery orifice) of a coronary artery (e.g., left coronary artery). Therefore, after the distal end of the guiding catheter is engaged with a branched portion of the target blood vessel, a treatment catheter inside the guiding catheter is protruded from the distal end of the guiding catheter, and the treatment catheter and a guide wire are fed to the stenosis located at a deep part (a distal-side portion) of the coronary artery.

Since the coronary artery ranging from the coronary artery orifice to the stenosis is thin (small in diameter) and winding, however, it is difficult for, particularly, a balloon part or a stent part of the treatment catheter to pass through the thin winding parts of the coronary artery. To smoothly advance the treatment catheter through these parts to the stenosis involves much difficulties, and imposes a heavy burden on the patient. Besides, even if an attempt to feed a guiding catheter into the vicinity of the stenosis located at a deep part of the coronary artery is made for avoiding such troubles, the guiding catheter having a certain degree of rigidity collides against the blood vessel wall in the course of passing through the coronary artery, thereby being bent unwillingly (see FIG. 15). As a result, the intended insertion into the deep part of the coronary artery becomes impossible ("stuck" state (failure in passage)).

In view of this, the catheter 1 as a guiding catheter is so designed that, after engaged alone, it can be fed further into the vicinity of the stenosis located in the deep part of the coronary artery and, in this condition, it permits the treatment catheter and the guide wire to be fed to the stenosis (see, for example, FIGS. 10 and 11). Consequently, the above-mentioned troubles can be solved. Here, "to engage" means to carry the guiding catheter to the coronary artery orifice of the coronary artery having a stenosis (target site), then to insert (engage) a distal portion of the guiding catheter into (with) the coronary artery orifice, and thereby to immobilize the guiding catheter in that position, prior to insertion of the treatment catheter. Besides, "alone" means that the catheter 1 is not used together with other guiding catheter (or support tube to be used for the purpose of guiding) but is used singly as a guiding catheter.

The catheter 1 is composed of a catheter body 3, a soft tip 4 which is rich in flexibility and provided on the distal side of the catheter body 3, and a hub 5 provided on the proximal side of the catheter body 3.

The catheter body 3 is composed of a flexible tube, and is provided in a substantially central portion thereof with a lumen 37 extending over the whole length of the catheter body 3. The lumen 37 is opening at the distal end of the soft tip 4.

Incidentally, the outside diameter ø d₁ of the catheter body 3 is set to be 1.4 to 1.6 mm, preferably 1.4 to 1.5 mm, more preferably 1.42 to 1.48 mm, and further preferably 1.43 mm (see FIGS. 2 and 3). Besides, the inside diameter ø d₂ of the catheter body 3 is preferably 1.2 to 1.5 mm, more preferably 1.24 to 1.4 mm (see FIGS. 2 and 3). With the outside diameter ø d₁ and the inside diameter ø d₂ of the catheter body 3 set in such numerical value ranges, the catheter 1 can be used satisfactorily as a guiding catheter.

In addition, while the outside diameter ø d₁ of the catheter body 3 is substantially the same as those of ordinary angiography catheters and microcatheters, the inside diameter ø d₂ is different from those of the angiography catheters and microcatheters in that it permits a treating device (balloon or stent) to be inserted through the catheter body 3 to a lesion for treating the lesion.

The distal end of a guiding catheter is not oriented (guided) by the flow of bloodstream. Therefore, the guiding catheter is different from a bloodflow guided catheter in that its distal end is not carried to a target site by bloodflow.

As shown in FIGS. 2 and 3, the tube composing the catheter body 3 is composed of a laminate obtained by laminating three layers, namely, an inner layer 34, an outer layer 35 and a reinforcing material layer 36 located therebetween.

The outer layer 35 includes a first region 351, a second region 352 located on the proximal side relative to the first region 351, a third region 353 located on the proximal side relative to the second region 352, and a fourth region 354 located on the proximal side relative to the third region 353. The third region 353 is lower in rigidity (flexural rigidity (bending stress), torsional rigidity), or more flexible, than the fourth region 354. In addition, the second region 352 is lower in rigidity than the third region 353. Besides, the first region 351 is lower in rigidity than the second region 352. This configuration ensures that the catheter body 3 is gradually lowered in rigidity, or increasing in flexibility, along the distal direction. Consequently, at the time of insertion into a blood vessel, the catheter body 3 can be inserted into the blood vessel more safely, while sufficiently securing pushability and torque transmission performance in the distal direction. Incidentally, while the catheter body 3 is configured here so as to be gradually lowered in rigidity along the distal direction, a configuration may be adopted in which the rigidity is substantially unvaried (the same) along the longitudinal direction.

Incidentally, let the flexural rigidity of a proximal-side portion (proximal portion 31) of the catheter body 3 be σ₁, then the flexural rigidity σ₁ is not particularly limited, but it is preferably, for example, 49.03 to 245.17•10⁻³ N (5 to 25 gf), more preferably, 98.07 to 196.13•10⁻³ N (10 to 20 gf). Herein, the "flexural rigidity" is a stress (measurable by autograph) measured by a method in which after the proximal portion side of the catheter body 3 is immersed in 37°C warm water for at least 30 min, the catheter body 3 is placed on a stainless steel jig in which two fulcrums having a height of 5 mm are arranged with a fulcrum-to-fulcrum distance of 45 mm, in 37°C warm water, and a central portion of the catheter body 3 is pressed by a pressing tool having a radius of 5 mm at a rate of 5 mm/min and by an amount of 3 mm (see FIG. 17).

In addition, let the flexural rigidity of a distal-side portion (curved part 33) of the catheter body 3 be σ₃, then the flexural rigidity σ₃ is preferably lower than the flexural rigidity σ₁ by 49.03 to 196.13•10⁻³ N (5 to 20 gf), more preferably by 68.65 to 147.10•10⁻³ N (7 to 15 gf). With the difference between the flexural rigidity σ₃ and the flexural rigidity σ₁ set within such a numerical value range, the catheter body 3 has little difference (a relatively small difference) in flexural rigidity between its distal-side portion and its proximal-side portion. As a result, the catheter body 3 can have appropriate rigidity (flexibility) over the whole part thereof, so that the catheter body 3 can be inserted to the coronary artery orifice easily and smoothly, and the engagement with the coronary artery orifice can be performed easily. In addition, safety for the blood vessel can be secured at the time of advancing the curved part 33 of the catheter body 3 inside the coronary artery toward the target site (stenosis).

Let the crushing strength of a proximal-side portion of the catheter body 3 be σ₂ [gf], then the ratio σ_{1/}σ₂ is not particularly limited, but it is preferably, for example, not more than 0.04, more preferably in the range of from 0.01 to 0.03. Herein, the "crushing strength" is a pressing force (strength) (measurable by autograph) measured by a method in which after the catheter body 3 is immersed in 37°C warm water for at least 30 min, the catheter body 3 is crushed in 37°C warm water by use of a pressing tool having a rectangular tip (see FIG. 18).

With the ratio σ_{1/}σ₂ set in such a numerical value range, it is ensured that, for example, when the catheter body 3 is inserted into a coronary artery and passed through a curved portion in the coronary artery, unwilling deformation (kinking (bending), twisting, crushing, or the like) of the proximal-side portion of the catheter body 3 can be prevented from occurring. Accordingly, steerability and safety at the time of insertion can be secured sufficiently.

Examples of materials for forming the first region 351, the second region 352, the third region 353 and the fourth region 354 include various thermoplastic elastomers based on styrene, polyolefin, polyurethane, polyester, polyamide, polybutadiene, trans-polyisoprene, fluoro-rubber, chlorinated polyethylene, or the like, which may be used either singly or in combination (polymer alloy, polymer blend, laminate, etc.) of two or more of them. By appropriately selecting the materials, for example, the catheter body 3 can be configured to be gradually lowered in rigidity along the distal direction or to be substantially unvaried (the same) in rigidity along the longitudinal direction.

In addition, preferred examples of these thermoplastic elastomers include polyester elastomers, polyamide elastomers, and polyurethane elastomers, which may be used either singly or in combination {polymer alloy, polymer blend, laminate, etc.) of two or more of them. By use of such materials, the catheter body 3 can be configured to have flexibility and anti-kinking property, specifically, appropriate rigidity.

As shown in FIG. 5, the surface (outside surface) 355 of the outer layer 35 is provided with a multiplicity of recesses 356. This ensures that when the catheter body 3 is inserted into a blood vessel, the area of contact of the surface 355 of the outer layer 35 of the catheter body 3 with the blood vessel wall is comparatively small. As a result, sliding resistance (friction) generated between the blood vessel wall and the surface 355 of the outer layer 35 of the catheter body 3 can be reduced, in other words, slidability of the catheter body 3 is enhanced. Accordingly, the curved part 33 can be assuredly inserted to a target site in the blood vessel (see FIGS. 10 to 14).

In addition, each of the recesses 356 is gradually increased in width (the length in the left-right direction in FIG. 5) toward the side of the surface 355; in other words, the recesses 356 are conical or bowl-like in shape.

The average of values of depth p (maximum depth; surface roughness Ry (the maximum height Ry defined in JIS B 0601-1994)) of the recesses 356 is not particularly limited, and it is preferably, for example, not more than 10 µm, more preferably 3 to 8 µm. Such recesses 356 are present in a resin layer (the outer layer 35) at positions between reinforcing materials 361 (or intersections of the reinforcing material 361).

Since the blood vessel wall is flexible, part of the blood vessel wall may enter into the recesses 356. When the average depth is within the above-mentioned numerical value range, it is ensured that even if part of the blood vessel wall has entered into the recesses 356, the part of the blood vessel wall is securely prevented from making contact with inside surfaces of the recesses 356, so that the contact area between the blood vessel wall and the outer layer 35 is prevented from being increased. In addition, the recesses 356 can be prevented from penetrating the outer layer 35 completely to reach the reinforcing material layer 36 or the inner layer 34. Consequently, the functions of the individual layers can be sufficiently displayed respectively.

Besides, the surface 355 of the outer layer 35 at the curved part 33 (distal portion) of the catheter body 3 is preferably coated, at least at part thereof, with a swellable coating. The coating is a coating which exhibits swellability upon contact with blood (liquid). The coating can be carried out by a method in which, for example, a commonly used gel such as PVA, or a maleic anhydride polymer, an acrylic polymer or the like is applied to the surface 355 of the outer layer 35.

Where the surface 355 of the outer layer 35 is provided thereon with the swellable coating, the (outside diameter of the curved part 33 increases upon contact of the curved part 33 with blood. As a result, the surface 355 of the curved part 33 is put into secure contact with, for example, a coronary artery orifice, so that the curved part 33 is engaged with the coronary artery orifice more assuredly.

The material composing the inner layer 34 is not particularly limited. Preferably, however, the inner layer 34, at least its portion coming into contact with a medical long body such as a guide wire 13 and a balloon catheter 15 at the time of inserting the medical long body into its lumen 37 (into the catheter body 3), is formed from a low-friction material. This ensures that the medical long body can be moved in the longitudinal direction or rotated relative to the catheter body 3 under a smaller sliding resistance, which contributes to enhancement of steerability.

Examples of the low-friction material include various resin materials such as polyamides, polyether polyamides, polyester polyamides, polyesters (polyethylene terephthalate, polybutylene terephthalate, polyethylene naphthalate, etc.), polyurethane, flexible polyvinyl chloride, ABS resin, AS resin, fluororesins such as polytetrafluoroethylene, and so on.

The reinforcing material layer 36 has the reinforcing material 361 for reinforcing the catheter body 3. Examples of the reinforcing material 361 include those composed of linear material, and those composed of net-like material.

Examples of the reinforcing material composed of linear material include spirally formed ones. The linear material is composed, for example, of any of various metals, hard resins or the like. Specific examples include those obtained by crushing stainless steel wires into a flat shape and then forming the flat-shaped wires into a spiral shape or those (braids) obtained by braiding (intersecting) the flat-shaped wires, in order that the catheter body 3 will be small in material thickness in the radial direction. Besides, in the case of a braid of linear materials, the angle θ of the reinforcing materials 361 against the center axis of the catheter body 3 is preferably 65 to 75 degrees, more preferably 69 to 72 degrees (see FIG. 4).

The provision of such a reinforcing material layer 36 makes it possible to secure sufficient rigidity and strength, without increasing the wall thickness of the catheter body 3, that is, while setting the inside diameter (the diameter of the lumen 37) at a comparatively large value. As a result, a catheter 1 is obtained which permits an inner catheter with a comparatively large outside diameter to be inserted therein and therethrough, is excellent in pushability and torque transmission performance, and is insusceptible to unwilling kinking.

Incidentally, the number of the layers composing the catheter body 3, the materials constituting the layers, the presence or absence of the reinforcing material 361, etc. may differ along the longitudinal direction of the catheter body 3. For instance, a distal-side portion (e.g., the curved part 33) of the catheter body 3 may have a reduced number of layers or be formed by use of a more flexible material, so as to be more flexible.

The insertion of the catheter 1 into a living body is carried out while checking its position under radioscopy. Therefore, the material composing the outer layer 35 preferably has a radiopaque material (radioscopic contrast medium) blended therein. Examples of the radiopaque material which can be used here include barium sulfate, bismuth oxide, and tungsten. Further, the proportion of the radiopaque material based on the material composing the outer layer 35 is preferably 30 to 80 wt%.

Besides, such a radiopaque material may not necessarily be present over the whole length of the catheter body 3. The radiopaque material may be present only in part of the catheter body 3, for instance, only in the curved part 33, or only in the soft tip 4.

In addition, the above-mentioned configuration of the inner layer 34 wherein its portion brought into contact with the medical long body is formed from the low-friction material is not limitative; for instance, the inner layer 34 may entirely be formed from the low-friction material.

When viewed along the longitudinal direction, the catheter body 3 as above can be divided into a proximal portion 31 and an intermediate portion 32 both extending substantially rectilinearly, and the curved part (distal portion) 33 which extends further along the distal direction from the intermediate portion 32 and has a desired curved shape, in this order from the proximal side. The curved part 33 of the catheter body 3 is curved into a desired shape which is suitable for that portion of a left coronary artery, a right coronary artery or the like into which the curved part 33 is to be inserted. Especially, the curved part 33 has such a shape that an operation (engaging operation) for engaging the curved part 33 with a coronary artery orifice (a branched portion of a blood vessel) is easy to carry out or such a shape that the engaged state of the curved part 33 engaged with the coronary artery orifice can be maintained more assuredly. Such a shape is not particularly limited. Examples of the shape include a shape (a hooked shape (J-shape)) as shown in FIG. 1 in which the curved part 33 is curved only toward one direction. Besides, such a shape contributes also to spreading (deformation) of the curved part 33 so that the portion, on the side of the soft tip 4, of the curved part 33 becomes farther from the intermediate portion 32. This enables the curved part 33 to be easily deformed from the engaged state (the state shown in FIG. 9) to a rectilinear state (the state shown in FIG. 10).

As shown in FIG. 1, the curved part 33 can be divided into two increasing curvature portions 311 where the curvature increases toward the distal end, and two decreasing curvature portions 312 where the curvature decreases. The two increasing curvature portions 311 and the two decreasing curvature portions 312 are alternately arranged along the longitudinal direction of the catheter body 3. The curved part 33 with such a configuration promises easy engagement thereof and can be deformed from the engaged state to the rectilinear state more easily.

Of the first region 351, the second region 352, the third region 353 and the fourth region 354 described above, at least the first region 351 is preferably formed as the curved part 33. In addition, the curved part 33 is preferably formed within the range of 300 mm from the distal end of the catheter 1.

In the catheter body 3 configured as above, the average total thickness t₁ of the inner layer 34, the outer layer 35 and the reinforcing material layer 36 in the curved part 33 is set in the range of 60 to 100 µm, preferably 65 to 95 µm, more preferably 70 to 90 µm, and further preferably 75 to 85 µm. Besides, the average minimum thickness t₂ of the outer layer 35 in the curved part 33 is set in the range of 8 to 30 µm, preferably 8 to 25 µm, and more preferably 8 to 20 µm. Here, "the minimum thickness of the outer layer 35" means the thickness of those portions of the outer layer 35 in which the reinforcing material layer 36 is embedded (minimum thickness portions 357 shown in FIGS. 2 and 3), specifically, the distance from an outermost periphery 362 of the reinforcing material layer 36 to the surface 355 of the outer layer 35.

More in detail, "the minimum thickness" is the thickness of those portions of the outer layer 35 at which the thickness of the outer layer 35 formed from a resin is the smallest. Specifically, in the cross section of the catheter shown in FIG. 3, on an extension line on a line interconnecting an edge of the section of the reinforcing material 361 close to the surface 355 of the outer layer 35 in the radial direction (a corner of the section close to the surface 355 of the outer layer 35) and the center of the catheter, the distance between the edge and the surface 355 of the outer layer 35 as measured along the straight line passing through the edge and the surface 355 of the outer layer 35, is the minimum thickness. In the case where the catheter 1 has a braid composed for example of sixteen reinforcing materials 361, the average of the distances between two edges each of the sections of an 8-membered set of reinforcing materials 361 on the side of the surface 355 of the outer layer 35 in the radial direction and the surface 355 of the outer layer 35 (the values of the above-mentioned minimum thickness), is the average minimum thickness t₂.

Incidentally, for measuring the thicknesses on section such as the average total thickness and the minimum thickness, those methods which are commonly used can be used. For instance, the periphery of the catheter as an object of measurement is fixed with a curable resin such as an epoxy resin, and the body resulting upon curing of the resin is sliced so that a desired section can be obtained. For example, the body is sliced along a direction perpendicular to the longitudinal axis of the catheter, and the resulting section is observed under a stereoscopic microscope, a CCD image sensor or the like, whereby the desired thickness can be measured.

Meanwhile, in the guiding catheter, its distal portion (the curved part 33) must have a certain.degree of rigidity so that the above-mentioned engagement can be performed easily and assuredly. The rigidity of the guiding catheter is needed also for enhancement of pushability and torque transmission performance in the distal direction. Besides, on the other hand, the distal portion of the guiding catheter must have flexibility such that, during an operation of inserting the guiding catheter, the guiding catheter can be smoothly advanced safely without damaging the blood vessel while selecting a branch of the blood vessel and following the curvature of the blood vessel. Thus, the guiding catheter is demanded to have mutually contradictory characteristics.

In view of this, in the catheter 1 (the catheter body 3), the above-mentioned numerical value ranges are specified for the curved part 33 so as to fulfill the mutually contradictory characteristics.

If the average total thickness t₁ and the average minimum thickness t₂ are below the lower limits of the above-mentioned numerical value ranges, the total thickness of the wall portion (tube wall) of the catheter body 3 is so small that only flexibility of the catheter body 3 can be secured whereas its rigidity is lowered conspicuously. On the other hand, if the average total thickness t₁ and the average minimum thickness t₂ are above the upper limits of the above-mentioned numerical value ranges, the total thickness of the wall portion (tube wall) of the catheter 3 is so large that only rigidity of the catheter body 3 can be secured whereas its flexibility is poor. In this case, further, the inside diameter ø d₂ is small, which leads to a demerit that there is a restriction imposed on the outside diameter of the treatment catheter to be inserted in the catheter 1.

In the catheter 1, the curved part 33 has appropriate rigidity and flexibility, since the average total thickness t₁ and the average minimum thickness t₂ in the curved part 33 are specified to be within the above-mentioned numerical value ranges. In other words, the catheter 1 is an ideal guiding catheter. As a result, the engagement can be assuredly achieved with the curved part 33. In addition, at the time of pushing the catheter 1 forward from the engaged state, the curved part 33 is deformed according to the curved shape of the blood vessel, so that the operation (pushing-in operation) can be carried out easily and assuredly. Thus, the catheter 1 can be inserted to a target site (stenosis) in a blood vessel through a simple operation and in a safe and assured manner.

Besides, in the curved part 33, the ratio d₁/t₂ between the average minimum thickness t₂ of the outer layer 35 and the outside diameter ø d₁ of the catheter body 3 is not particularly limited, but it is preferably in the range of, for example, from 50 to 250, more preferably from 70 to 200. It has been verified that, when the ratio d_{1/}t₂ is within such a range, the rigidity of the catheter body 3 of a catheter 1 having an outside diameter ø d₁ of 1.4 to 1.5 mm and its deformability in a coronary artery are both attained satisfactorily. Similarly, the same result could also be obtained with catheters 1 having an outside diameter ø d₁ of 1.6 to 1.75 mm, and further with catheters 1 having an outside diameter ø d₁ of not less than 1.95 mm.

Incidentally, the configuration of the catheter, body 3 in which the curved part 33 satisfies the above-mentioned conditions specified for the curved part 33 is not limitative; for example, a configuration is preferable in which the intermediate portion 32 and the proximal portion 31 also satisfy the above-mentioned conditions.

As shown in FIGS. 1 and 2, the soft tip 4 is further connected to the distal end of the curved part 33 (the catheter body 3). The soft tip 4 is more flexible than the catheter body 3, and its distal end preferably has a rounded shape (such a shape as to be able to securely prevent damages to tissues) (see FIG. 2). Where such a soft tip 4 is provided, the catheter 1 can be moved smoothly and safely even in a blood vessel which is curved, bent and branched.

Examples of the material for forming the soft tip 4 include various rubber materials such as natural rubber, isoprene rubber, butadiene rubber, chloroprene rubber, silicone rubber, fluororubber, styrene-butadiene rubber, etc. and various thermoplastic elastomers based on styrene, polyolefin, polyurethane, polyester, polyamide, polybutadiene, trans-polyisoprene, fluororubber, chlorinated polyethylene or the like.

In addition, the material composing the soft tip 4 may have the above-mentioned radiopaque material (radioscopic contrast medium) blended wherein.

Besides, the length of the curved part 33 is preferably 3 to 50 times, more preferably 5 to 20 times, the length of the soft tip 4. Specifically, the length of the soft tip 4 is set to be preferably about 0.5 to 3 mm, more preferably about 1 to 2 mm.

The hub 5 is mounted (secured) to the proximal end of the catheter body 3. The hub 5 is formed therein with an inner cavity communicating with the lumen 37. The inner cavity has an inside diameter approximately equal to the inside diameter of the lumen 37, so that it is continuous with the inside surface of a proximal portion of the lumen 37, without generating a step or the like therebetween.

Long bodies (linear bodies) such as a guide wire, catheters (e.g., balloon catheter, stent transport catheter or vasodilation catheter for use in PTCA), an endoscope, an ultrasonic probe, a temperature sensor, etc. can be inserted or pulled out through the hub 5 as above. In addition, the hub 5 can be connected with other equipment at the time of, for example, blood pressure measurement or the like.

Besides, at a portion where the catheter body 3 and the hub 5 are interconnected, a covering material (anti-kinking protector) 51 composed of an elastic material is provided. As a result, sharp bending (kinking) can be prevented from occurring in the vicinity of the joint portion.

Now, an example of the method of using the catheter 1 will be described in detail below.

As shown in FIG. 7, by the Seldinger technique, a catheter introducer 11 is made to puncture an artery (introduction site) 14 such as, for example, a right femoral artery, a right brachial artery or a right radial artery, and the catheter body 3 (the catheter 1) is inserted into a sheath 12 of the catheter introducer 11. In this instance, a guide wire 13 is preliminarily inserted in the lumen 37 of the catheter body 3. Then, while letting the guide wire 13 precede, the distal end of the catheter body 3 is inserted through a distal opening 121 of the sheath 12 into the artery 14.

Next, the catheter body 3 is gradually advanced while letting the guide wire 13 precede in the direction of arrow in FIG. 7, toward a site (e.g., a right coronary artery, a left coronary artery or the like) into which the curved part 33 of the catheter body 3 is to be inserted and at which the curved part 33 is to be set indwelling. In this case, in order to smoothly pass the curved part 33 though a curved portion (bent portion) of the blood vessel or in order to achieve proper selection of a branch in the blood vessel, operations including an appropriate combination of letting-in and letting-out of the guide wire 13 relative to the catheter 1 as well as forward movement, backward movement and rotation of the catheter 1 are carried out.

Hereinafter, operations (procedures) at the time of inserting the catheter 1 into a left coronary artery 101 will be described. Incidentally, these operations are all carried out while checking the position and attitude of the catheter body 3 under radioscopy.

In the condition where a femoral artery is punctured with the catheter introducer 11 and the guide wire 13 is inserted in the lumen 37 of the catheter 1, the catheter 1 is inserted into the catheter introducer 11. Incidentally, in the condition where the guide wire 13 is protruded from the distal end (the distal opening 41) of the catheter body 3, the curved part 33 of the catheter 1 is substantially rectilinear due to the rigidity of the guide wire 13.

After the catheter 1 is thus inserted through the catheter introducer 11 into the femoral artery as above, the curved part 33 of the catheter body 3 is advanced through an abdominal aorta 103 to an ascending aorta 100, while letting the guide wire 13 precede. At the time when the soft tip 4 having fed past an aortic arch 109 is located at a position about 10 cm above a left coronary artery orifice 106 of the left coronary artery 101, the advancement of the catheter 1 is stopped, and the guide wire 13 is pulled out so that the shape of the curved part 33 returns to the original curved shape (natural state).

Subsequently, while checking the distal end position of the catheter 1 (the position of the soft tip 4), the catheter 1 is slowly pushed forward, whereon the curved part 33 is moved downward while keeping contact with a left inside wall 104 of the ascending aorta 100, and is inserted into the left coronary artery orifice 106
(branched portion) (see FIG. 8). The shape of the curved part 33 in this instance is such a shape as to permit easy engagement.

Incidentally, in the case where the curved part 33 of the catheter 1 is oriented in a direction opposite to the left coronary artery orifice 106, the catheter 1 is slightly rotated counterclockwise to orient the curved part 33 toward the left coronary artery orifice 106, and is slowly pushed forward while keeping this orientation. As a result, the curved part 33 is easily inserted into the left coronary artery orifice 106, and is engaged in that condition (see FIG. 8).

After the curved part 33 (the soft tip 4) is inserted into and engaged with the left coronary artery orifice 106 by the above-mentioned operations, a connector of a radiopaque contrast agent injecting instrument (not shown) is connected to the hub 5, and the radiopaque contrast agent is injected. The radiopaque contrast agent thus injected passes through the hub 5 and through the lumen 37, to be jetted from the distal opening 41 of the soft tip 4 into the left coronary artery 101. As a result, a stenosis (diseased part) 110 present in the left coronary artery 101 is radioscopically imaged, and its position is determined.

Next, while keeping the engaged state, the connector of the radiopaque contrast agent injecting instrument is detached from the hub 5. Thereafter, the catheter 1 (the curved part 33) is pushed forward in the distal direction (toward the distal side) further from the left coronary artery orifice 106 (see FIG. 9). When the soft tip 4 has reached the proximal vicinity of the stenosis 110 as the target site, the advancement of the catheter 1 is once stopped (see FIG. 10). In this case, in order to smoothly pass the soft tip 4 through curved or bent portions of the left coronary artery 101 or in order to attain proper selection of a branch in the left coronary artery, operations including an appropriate combination of forward movement, backward movement and rotation of the catheter 1 are carried out. The curved part 33 can be deformed according to the shape of the portion (the left coronary artery 101) on the distal side relative to the left coronary artery orifice 106, since it has appropriate flexibility as above-mentioned. As a result, the above-mentioned pushing-in operation can be carried out assuredly.

Subsequently, the guide wire 13' is again inserted into the lumen 37 of the catheter body 3 via the hub 5, and the distal end of the guide wire 13' is protruded from the distal opening 41 of the soft tip 4.

Next, a treatment catheter such as a balloon catheter 15 for PTCA is inserted into the lumen 37 of the catheter 1 via the hub 5, arid a distal portion 151 of the balloon catheter 15 for PTCA is protruded from the distal opening 41. In this instance, the guide wire 13' is in the state of being inserted in a lumen of the PTCA balloon catheter 15, and the distal end of the guide wire 13' is protruded from the distal end of the balloon catheter 15.

The balloon catheter 15 is thus inserted through the lumen 37 of the catheter body 3. Therefore, the distal portion of the balloon catheter 15 can be guided to a target site smoothly and safely, without generating a stuck state (failure in passage) of a balloon 152 or the like of the balloon catheter 15 in the thin and winding left coronary artery 101, as compared with the case in which the balloon catheter 15 is directly inserted into and advanced inside the left coronary artery 101 via the left coronary artery orifice 106. In addition, such operations can be carried out in a shorter time, so that the burden on the patient can be alleviated.

Further, while letting the guide wire 13' precede, the balloon part of the balloon catheter 15 is advanced to the stenosis 110, and the balloon 152 is inflated to apply a dilating treatment to the stenosis 110 (see FIG. 11).

In the case where a stenosis 110 exists in a right coronary artery 102 and a dilating treatment is to be applied thereto, as shown in FIG. 12, also, a procedure similar to the above-mentioned and suited to the situation can be carried out (see FIG. 12).

As shown in FIG. 13, insertion of the catheter 1 into a heart (in the condition shown in FIG. 13, intro the right coronary artery 102) via a brachiocephalic trunk 107 can also be performed. In these cases, also, a procedure similar to the above-mentioned can be carried out (the same applies also to the left coronary artery 101). Further, the same applies also to the cases.where the catheter 1 is inserted via a left common carotid artery 108 or a left subclavian artery 105, instead of the brachiocephalic trunk 107.

Besides, while the cases where the balloon catheter 15 for PTCA is used as the treatment catheter have been described above, the treatment catheter is not limited thereto. For example, also in the case of using a stent transport catheter 17 for transporting a stent (a tubular medical device) 16 and releasing it in a stenosis 110 in the right coronary artery 102, as shown in FIG. 13, the procedure is substantially the same as above-mentioned (the same applies also to the left coronary artery 101). In this case, the inside diameter of the stent 16 is preferably 2.2 to 3.0 mm, more preferably 2.6 to 2.8 mm.

In addition, also in the case where a stent has already been set indwelling in a blood vessel, as shown in FIG. 14, the catheter can be inserted past the stent so as to apply a dilating treatment to a stenosis 110 located beyond the stent.

### <Second Embodiment>

FIG. 6 is a longitudinal sectional view of a distal portion of a catheter (second embodiment) according to the present invention.

While a second embodiment of the catheter according to the present invention will be described below referring to this figure, the description is centered on differences of this embodiment from the above-described embodiment, and descriptions of the same items as above will be omitted.

This embodiment is the same as the first embodiment above, except for a difference in the configuration of the soft tip.

In a catheter 1A shown in FIG. 6, the soft tip 4 is formed from the same material as that of the outer layer 35. This enables the soft tip 4 to be formed integrally with the outer layer 35. In addition, for the purpose of enhancing radioscopic imageability and reinforcing the soft tip 4, a distal portion (a portion ranging to 2 to 3 mm from the distal opening 41) of the catheter body 3 may be enlarged in outside diameter ø d₁. In this case, the inside diameter ø d₂ of the catheter body 3 is left unchanged.

As has been described above, the distal end (the distal opening 41) of the catheter 1 (the same applies also to the catheter 1A) is engaged with the inside of a coronary artery orifice (a left coronary artery orifice 106), then a guide wire 13' is inserted, the distal end of the catheter 1 is advanced inside a coronary artery (a left coronary artery 101) along the preceding guide wire 13', and the distal end of the catheter 1 is guided toward the distal side in the coronary artery.

The distal end of the catheter 1 is advanced past an already treated part (a part where a stent 16 is left indwelling) toward the further distal side. Specifically, the distal end of the catheter 1 is advanced through the inside of the stent 16, which is left indwelling in the coronary artery, so as to be located on the distal side of the stent 16. Then, a treatment catheter (a balloon catheter, a stent transport catheter, or a vasodilation catheter) can be protruded from the distal end of the catheter 1 and can be guided to and located in a target site. The treatment catheter can be guided to the target site smoothly and safely, without being caught on the stent 16 which is already left indwelling. Such operations can also be conducted in a short time, so that the burden on the patient is lessened. The treatment catheter protruded from the distal end of the catheter 1 having passed through the inside of the already indwelling stent 16 is advanced to the target site, and a dilating treatment is applied to a stenosis part (stenosis 110) located on the distal side of the indwelling stent 16 (see FIG. 14).

A wall part of the catheter body 3 as a whole is higher in rigidity than the soft tip 4, since it includes the inner layer 34, the outer layer 35 and the reinforcing material layer 36. In other words, the soft tip 4 is a part which is more flexible than the catheter body 3.

In addition, since a boundary portion between the soft tip 4 and the catheter body 3 (the outer layer 35) in the catheter 1A is smoothly varied in shape, the boundary portion can be more suitably deformed according to the curved shape of a blood vessel during passage in the blood vessel. This enables the curved part 33 to be assuredly inserted to a target site through a simple operation.

While the embodiments of the catheter of the present invention shown in the drawings have been described above, the invention is not limited to the embodiments. The components of the catheter can be replaced by those of arbitrary configurations which can exhibit functions identical or equivalent to the above-mentioned. Besides, arbitrary structures may be added.

In addition, the use for the catheter according to the present invention is not particularly limited. The catheter of the invention is applicable also to guiding catheters for guiding, for example, an atherectomy catheter, an ultrasound catheter or the like, in addition to the above-mentioned catheters. Besides, that part of a living body into which the catheter is to be inserted is, naturally, not limited to the coronary artery.

### Examples

Now, specific working examples of the present invention will be described below.

### (Example 1)

On a linear material obtained by forming a 10 µm-thick PTFE layer on a copper wire having a diameter of 1.27 mm, stainless steel flat plate reinforcing wires (in a 16-membered set) of 80 µm in width and 25 µm in thickness are wound by braiding at an interval of 0.1 mm.

The reinforcing wires are cut at both ends thereof, and various materials are provided on the linear material and the copper wire, starting from the distal end side. Specifically, a short tube of 1 mm in length composed of a polyester elastomer resin having a Shore D hardness (American Society for Testing and Materials standard ASTM-D2240) of 44 and containing 60 wt% of tungsten as a radiopaque material and 1 wt% of a pigment is provided on a most distal portion of the linear material. Next, same-length short tubes of polyester elastomer resins different in hardness are sequentially provided on the linear material in four stages so that the hardness is increased stepwise. Finally, a polyester elastomer resin tube having a Shore D hardness of 68 is provided on an about 1000 mm remaining portion on the proximal side of the linear material. End portions of the tubes are made to abut on each other, and then the whole part of the assembly is covered with a heat-shrinkable tube, followed by heating to effect heat fusing. Thereafter, the heat-shrinkable tube is peeled off, and the copper wire is drawn out. By such a method, a tube provided therein with a penetrating lumen and having an outside diameter ø d₁ of 1.43 mm, an inside diameter ø d₂ of 1.27 mm, a d₁/d₂ ratio of 1.13 and a length of 1070 mm was obtained.

The tube obtained as above was subjected to heating of a soft tip, which is the portion where the reinforcing wires and the inner layer are absent, in a mold so as to round the soft tip, thereby obtaining a catheter body.

A catheter portion ranging to 150 mm from the distal end was coated with a hydrophilic resin.

A core metal having a curved shape was inserted in the thus completed catheter body, and the assembly was heated in an oven and deformed, whereby a distal portion is formed into a Judkins left 4.0 shape (the numeral represents the curve size). Finally, a hub and an anti-kinking protector were attached to the proximal side of the catheter body, to obtain a 1.33 mm (4 Fr) guiding catheter.

The catheter had a material thickness (average total thickness t₁) of 80 µm, an average minimum thickness t₂ of the outer layer (resin layer) of 8 µm, a flexural rigidity σ₃ of a distal portion of 78.45•10⁻³ N (8 gf), a flexural rigidity σ₁ of a proximal portion of 166.71•10⁻³ N (17 gf), and a flexural rigidity difference between the proximal portion and the distal portion of 88.26•10⁻³ N (9 gf). In addition, the crushing strength σ₂ was 5.2 N (530 gf), the ratio σ₁/σ₂ was 0.031, and the maximum height Ry of surface roughness of the outer layer (measured by a contact type surface roughness tester SJ-400, produced by Mitutoyo Corporation, according to JIS B 0601-1994) was 4.0 μm.

### (Example 2)

On a linear material obtained by forming a 10 μm-thick PTFE layer on a copper wire having a diameter of 1.27 mm, stainless steel flat plate reinforcing wires (in a 16-membered set) of 80 μm in width and 25 μm in thickness are wound by braiding at an interval of 0.1 mm.

The reinforcing wires are cut at both ends thereof, and various materials are provided on the linear material and the copper wire, starting from the distal end side. Specifically, a short soft tip of 1 mm in length composed of a polyester elastomer resin having a Shore D hardness (American Society for Testing and Materials standard ASTM-D2240) of 44 and containing 60 wt% of tungsten as a radiopaque material and 1 wt% of a pigment is provided on a most distal portion of the linear material. Next, 3 mm short tubes of polyester elastomer resins different in hardness are sequentially provided on the linear material in three stages, and different-length tubes of polyester elastomer resins having a Shore D hardness of 57, a Shore D hardness of 68 and a Shore D hardness of 78 respectively are provided on the linear material. End portions of the tubes are made to abut on each other, and then the whole part of the assembly is covered with a heat-shrinkable tube, followed by heating to effect heat fusing. Thereafter, the heat-shrinkable tube is peeled off, and the copper wire is drawn out. By such a method, a tube provided therein with a penetrating lumen and having an outside diameter ø d₁ of 1.43 mm, an inside diameter ø d₂ of 1.27 mm, a d₁/d₂ ratio of 1.13 and a length of 1070 mm was obtained.

The tube obtained as above was subjected to heating of the soft tip, which is the portion where the reinforcing wires and the inner layer are absent, in a mold so as to round the soft tip, thereby obtaining a catheter body. A catheter portion ranging to 150 mm from the distal end was coated with a hydrophilic resin.

A core metal having a curved shape was inserted in the thus completed catheter body, and the assembly was heated in an oven and deformed, whereby a distal portion is formed into a Judkins left 4.0 shape (the numeral represents the curve size). Finally, a hub and an anti-kinking protector were attached to the proximal side of the catheter body, to obtain a 1.33 mm (4 Fr) guiding catheter.

The catheter had a material thickness (average total thickness t₁) of 80 µm, and an average minimum thickness t₂ of the outer layer (resin layer) of 8 μm. The flexural rigidity σ₃ of a distal portion was 78.45•10⁻³N (8 gf). The portion of Shore D hardness of 57 had a flexural rigidity of 117.68•10⁻³ N (12 gf) and a crushing strength of 4.4 N (450 gf); the portion of Shore D hardness of 68 had a flexural rigidity of 166.71•10⁻³ N (17 gf) and a crushing strength of 5.2 N (530 gf); and the proximal portion of Shore D hardness of 78 had a flexural rigidity σ₁ of 205.94•10⁻³ N (21 gf) and a crushing strength of 5.9 N (600 gf). The flexural rigidity difference between the proximal portion and the distal portion was 127.49•10⁻³ N (13 gf). The maximum height Ry of surface roughness of the outer layer (measured by a contact type surface roughness tester SJ-400, produced by Mitutoyo Corporation, according to JIS B 0601-1994) was 4.0 μm.

### (Example 3)

A catheter was produced in the same manner as in Example 1, except that a copper wire having a diameter of 1.25 mm was used. The ratio of outside diameter to inside diameter of the catheter was the same as that of a 2 mm (6 Fr) guiding catheter.

The catheter had a material thickness (average total thickness t1) of 90 μm, an average minimum thickness t₂ of the outer layer (resin layer) of 19 μm, a flexural rigidity σ₃ of a distal portion of 88.26•10⁻³ N (9 gf), a flexural rigidity σ₁ of a proximal portion of 176.52•10⁻³ N (18 gf), and a flexural rigidity difference between the proximal portion and the distal portion of 107.87•10⁻³ N (11 gf). In addition, the crushing strength σ₂ was 4.7 N (480 gf), the ratio σ₁/σ₂ was 0.038, and the maximum height Ry of surface roughness of the outer layer (measured by a contact type surface roughness tester SJ-400, produced by Mitutoyo Corporation, according to JIS B 0601-1994) was 3.4 µm.

A treatment device (balloon catheter or the like) passed through the inside of the catheter in Example 3 was easily passed through an about 5 mm-long rectilinear portion and an R15 curved portion (a curved portion with a radius of curvature of 15 mm) of a left coronary artery model shown in FIG. 21. However, when the distal end of the catheter in Example 3 was located in an R7.5 curved portion and the treatment device passed through the inside of the catheter was located in an R10 curved portion, the catheter of Example 3 gave a somewhat heavier feeling to hand when pushed or pulled, as compared with the catheters of Examples 1 and 2.

### (Comparative Example 1)

A guiding catheter was produced in the same manner as in Example 1, except that the outside diameter ϕd₁ obtained was 1.41 mm. The guiding catheter had a flexural rigidity of a proximal portion of 98.07•10⁻³ N (10 gf), a crushing strength σ₂ of 4.4 N (450 gf), an anti-kinking strength of 15 mm, and an average minimum thickness t₂ of 4 μm.

### (Comparative Example 2)

A guiding catheter was produced in the same manner as in Example 1, except that the outside diameter ø d₁ obtained was 1.49 mm. The guiding catheter had a flexural rigidity σ₁ of a proximal portion of 192.21•10⁻³ N (19.6 gf), a crushing strength σ₂ of 6.4 N (650 gf), an anti-kinking strength of 7 mm, and an average minimum thickness t₂ of 48 μm. The maximum height Ry of surface roughness (measured by a contact type surface roughness tester SJ-400, produced by Mitutoyo Corporation, according to JIS 1994) was 2.8 μm.

### (Comparative Example 3)

A guiding catheter was produced in the same manner as in Example 1, except that the Shore D hardness of the resin constituting a proximal portion was 57. The guiding catheter thus obtained had an outside diameter ø d₁ of 1.43 mm, a flexural rigidity σ₁ of the proximal portion of 107.87•10⁻³ N (11 gf), a crushing strength σ₂ of 4.4 N (450 gf), and an average minimum thickness t₂ of 10 μm.

### (Comparative Example 4)

A guiding catheter was produced in the same manner as in Example 1, except that the Shore D hardness of the resin constituting a proximal portion was 78. The guiding catheter thus obtained had an outside diameter ø d₁ of 1.43 mm, a flexural rigidity σ₁ of the proximal portion of 205.94•10⁻³ N (21 gf), a crushing strength σ₂ of 5.9 N (600 gf), and an average minimum thickness t₂ of 9 μm.

### (Comparative Example 5)

By using a polyester elastomer with a Shore D hardness of 60 and a stainless steel wire with a wire diameter of 50 μm, an angiography catheter having a distal portion in Judkins left JL 4.0 shape and having an outside diameter ø d₁ of 1.41 mm, an inside diameter ø d₂ of 0.97 mm and a Shore D hardness of 60 was produced. The catheter had a material thickness (average total thickness t₁) of 220 μm, a flexural rigidity σ₁ of a proximal portion of 176.52•10⁻³ N (18 gf), a crushing strength σ₂ of 9.2 N (935 gf), and an average minimum thickness t₂ of 120 μm.

### (Comparative Example 6)

By using a polyester elastomer with a Shore D hardness of 60 and a stainless steel wire with a wire diameter of 50 μm, an angiography catheter straight in its distal portion (with no curved shape) and having an outside diameter ø d1 of 1.41 mm, an inside diameter ø d₂ of 0.97 mm and a Shore D hardness of 60 was produced. The catheter had a material thickness (average total thickness t₁) of 220 μm, a flexural rigidity σ₁ of 176.52•10⁻³ N (18 gf), a crushing strength σ₂ of 9.1 N (925 gf), and an average minimum thickness t₂ of 120 μm.

### (Comparative Example 7)

On a linear material obtained by forming a 10 μm-thick PTFE layer on a copper wire having a diameter of 1.50 mm, stainless steel flat plate reinforcing wires (in a 16-membered set) of 135 μm in width and 30 μm in thickness are wound by braiding at an interval of 0.15 mm.

The reinforcing wires are cut at both ends thereof, and various materials are provided on the linear material and the copper wire, starting from the distal end side. Specifically, a short soft tip of 1 mm in length composed of a polyester elastomer resin having a Shore D hardness (American Society for Testing and Materials standard ASTM-D2240) of 35 and containing 68 wt% of tungsten as a radiopaque material and 2 wt% of a pigment is provided on a most distal portion of the linear material. Next, 3 mm short tubes of polyester elastomer resins different in hardness are sequentially provided on the linear material in four stages. Finally, a polyester elastomer resin tube having a Shore D hardness of 68 is provided on an about 300 mm remaining portion on the proximal side of the linear material. Finally, a polyester elastomer resin tube having a Shore D hardness of 78 is provided on an about 700 mm remaining portion on the proximal side of the linear material. End portions of the tubes are made to abut on each other, and then the whole part of the assembly is covered with a heat-shrinkable tube, followed by heating to effect heat fusing. Thereafter, the heat-shrinkable tube is peeled off, and the copper wire is drawn out. By such a method, a tube provided therein with a penetrating lumen and having an outside diameter ø d₁ of 1.73 mm, an inside diameter ø d₂ of 1.50 mm and a length of 1020 mm was obtained.

The tube obtained as above was subjected to heating of a distal soft tip part, which is the portion where the reinforcing wires and the inner layer are absent, in a mold so as to round the soft tip part, thereby obtaining a catheter body.

A core metal having a curved shape was inserted in the catheter body, and the assembly was heated in an oven and deformed, whereby a distal portion is formed into a Judkins left 4.0 shape (the numeral represents the curve size). Finally, a hub and an anti-kinking protector were attached to the proximal side of the catheter body, to obtain a 1.33 mm (4 Fr) guiding catheter.

The catheter had a material thickness (average total thickness t₁) of 115 μm, an average minimum thickness t₂ of the outer layer (resin layer) of 39 μm, a flexural rigidity σ₃ of a distal portion of 78.45•10⁻³ N (8 gf), a flexural rigidity σ1 of a proximal portion of 441.3•10⁻³ N (45 gf), and a flexural rigidity difference between the proximal portion and the distal portion of 362.85•10⁻³ N (37 gf). In addition, the crushing strength was 8.6 N (880 gf), and the ratio σ₁/σ₂ was 0.051. The catheter was hard at its distal portion, and when the catheter was inserted in a curved silicone tube of 3 mm in inside diameter imitating a left coronary artery, the catheter was not deformed along the silicone tube. In addition, when the catheter was inserted into an expanded stent, a bent catheter portion at 10 mm from the distal end made contact with the stent and deformed the stent, resulting in that the stent was caught on the distal end of the catheter.

### (Comparative Example 8)

On a linear material obtained by forming a 10 μm-thick PTFE layer on a copper wire having a diameter of 1.50 mm, stainless steel flat plate reinforcing wires (in a 16-membered set) of 127 µm in width and 25 µm in thickness are wound by braiding at an interval of 0.15 mm.

The reinforcing wires are cut at both ends thereof, and various materials are provided on the linear material and the copper wire, starting from the distal end side. Specifically, a short soft tip of 1 mm in length composed of a polyester elastomer resin having a Shore D hardness (American Society for Testing and Materials standard ASTM-D2240) of 38 and containing 60 wt% of tungsten as a radiopaque material and 1 wt% of a pigment is provided on a most distal portion of the linear material. Next, a short tube of 120 mm in length of a different-hardness polyester elastomer resin having a Shore D hardness of 44 and containing 30 wt% of tungsten as a raidopaque material, a short tube of 30 mm in length of a different-hardness polyester elastomer resin having a Shore D hardness of 44 and containing 30 wt% of tungsten as a radiopaque material, a short tube of 45 mm in length of a different-hardness polyester elastomer resin having a Shore D hardness of 52 and containing 30 wt% of tungsten as a radiopaque material, a short tube of 120 mm in length of a different-hardness polyester elastomer resin having a Shore D hardness of 52 and containing 30 wt% of tungsten as a radiopaque material, and a short tube of 150 mm in length of a polyester elastomer resin tube having a Shore D hardness of 68, were provided on the linear material.. Finally, a polyester elastomer resin tube having a Shore D hardness of 78 is provided on an about 700 mm remaining portion on the proximal side of the linear material. End portions of the tubes are made to abut on each other, and then the whole part of the assembly is covered with a heat-shrinkable tube, followed by heating to effect heat fusing. Thereafter, the heat-shrinkable tube is peeled off, and the copper wire is drawn out. By such a method, a tube provided therein with a penetrating lumen and having an outside diameter ø d₁ of 1.68 mm, an inside diameter ø d₂ of 1.50 mm and a length of 1250 mm was obtained.

The tube obtained as above was subjected to heating of a distal soft tip part, which is the portion where the reinforcing wires and the inner layer are absent, in a mold so as to round the soft tip part, thereby obtaining a catheter body.

When a core metal having a curved shape was inserted in the catheter body and the assembly was heated in an oven, the distal portion of the catheter body was little deformed. Finally, a hub and an anti-kinking protector were attached to the proximal side of the catheter body, to obtain a 1.67 mm (5 Fr) guiding catheter.

The catheter had a material thickness (average total thickness t₁) of 115 μm, an average minimum thickness t₂ of the outer layer (resin layer) of 28 μm, a flexural rigidity σ₃ of a distal portion of 68.65•10⁻³ N (7 gf), a flexural rigidity σ₁ of a proximal portion of 392.27•10⁻³ N (40 gf), and a flexural rigidity difference between the proximal portion and the distal portion of 323.62•10⁻³ N (33 gf). In addition, the crushing strength was 6.3 N (640 gf), and the ratio σ₁/σ₂ was 0.063. The catheter was flexible at its distal portion, and when the catheter was inserted in a curved silicone tube of 3 mm in inside diameter imitating a left coronary artery, the catheter was deformed along the silicone tube. In addition, when the catheter was inserted into an expanded stent, the catheter was smoothly passed through the stent. Then catheter could not be engaged with a branched portion of a coronary artery when used alone. In view of this, a 2 mm (6 Fr) guiding catheter was first inserted and engaged with the branched portion of the coronary artery, and then the catheter was passed inside the 2 mm (6 Fr) guiding catheter, before being inserted into the coronary artery.

### (Comparative Example 9)

On a linear material obtained by forming a 10 μm-thick PTFE layer on a copper wire having a diameter of 1.80 mm, stainless steel flat plate reinforcing wires (in a 16-membered set) of 110 μm in width and 35 μm in thickness are wound by braiding at an interval of 0.15 mm.

The reinforcing wires are cut at both ends thereof, and various materials are provided on the linear material and the copper wire, starting from the distal end side. Specifically, a short soft tip of 1 mm in length composed of a polyester elastomer resin having a Shore D hardness (American Society for Testing and Materials standard ASTM-D2240) of 29 and containing 68 wt% of tungsten as a radiopaque material and 4 wt% of a pigment is provided on a most distal portion of the linear material. Next, short tubes of 3 mm in length of polyester elastomer resins different in hardness are sequentially provided on the linear material in four stages. Finally, a polyester elastomer resin tube having a Shore D hardness of 78 is provided on an about 1000 mm remaining portion on the proximal side of the linear material. End portions of the tubes are made to abut on each other, and then the whole part of the assembly is covered with a heat-shrinkable tube, followed by heating to effect heat fusing. Thereafter, the heat-shrinkable tube is peeled off, and the copper wire is drawn out. By such a method, a tube provided therein with a penetrating lumen and having an outside diameter ø d₁ of 2.06 mm, an inside diameter ø d₂ of 1.80 mm and a length of 1020 mm was obtained.

The tube obtained as above was subjected to heating of the distal soft tip part, which is the portion where the reinforcing wires and the inner layer are absent, in a mold so as to round the soft tip part, thereby obtaining a catheter body.

When a core metal having a curved shape was inserted in the catheter body and the assembly was heated in an oven, whereby the distal portion of the catheter body was little deformed. Finally, a hub and an anti-kinking protector were attached to the proximal side of the catheter body, to obtain a 1.67 mm (5 Fr) guiding catheter.

The catheter had a material thickness (average total thickness t₁) of 130 μm, an average minimum thickness t₂ of the outer layer (resin layer) of 28 μm, a flexural rigidity σ₃ of a distal portion of 147.1•10⁻³ N (15 gf), a flexural rigidity σ₁ of a proximal portion of 686.47•10⁻³ N (70 gf), and a flexural rigidity difference between the proximal portion and the distal portion of 539.37•10⁻³ N (55 gf). In addition, the crushing strength was 10.6 N (1078 gf), and the ratio σ₁/σ₂ was 0.065. The catheter was hard at its distal portion, and, when it was attempted to insert the catheter into a curved silicone tube of 3 mm in inside diameter imitating a left coronary artery, the insertion was difficult to achieve due to the large outside diameter of the catheter, and the catheter was not deformed along the tube. Besides, when the catheter was inserted into an expanded stent, a bent catheter portion at 10 mm from the distal end made contact with the stent and deformed the stent, resulting in that the stent was caught on the distal end of the catheter.

### (Comparative Example 10)

A guiding catheter was produced in the same manner as in Example 1, except that a copper wire having a diameter of 1.17 mm was used. The guiding catheter had a flexural rigidity of a distal portion of 78.45•10⁻³ N (8 gf), a flexural rigidity of a proximal portion of 176.52•10⁻³ N (18 gf), a crushing strength σ₂ of 5.7 N (580 gf), and an average minimum thickness t₂ of 55 μm.

### (Comparative Example 11)

A guiding catheter was produced in the same manner as in Example 1, except that a copper wire having a diameter of 1.20 mm was used. The guiding catheter had a flexural rigidity of a distal portion of 88.26•10⁻³ N (9 gf), a flexural rigidity of a proximal portion of 186.33•10⁻³ N (19 gf), a crushing strength σ₂ of 5.4 N (550 gf), and an average minimum thickness t₂ of 43 μm.

### (Comparative Example 12)

A guiding catheter was produced in the same manner as in Comparative Example 5, except that the outside diameter ϕd1 obtained was 1.66 mm. The guiding catheter had a flexural rigidity of a distal portion of 78.45•10⁻³ N (8 gf), a flexural rigidity of a proximal portion of 264.78•10⁻³ N (27 gf), a crushing strength σ₂ of 6.9 N (700 gf), and an average minimum thickness t₂ of 4 μm.

### (Comparative Example 13)

A guiding catheter was produced in the same manner as in Comparative Example 5, except that a copper wire having a diameter of 1.88 mm was used. The guiding catheter had a flexural rigidity of a distal portion of 78.45•10⁻³ N (8 gf), a flexural rigidity of a proximal portion of 392.27•10⁻³ N (40 gf), a crushing strength σ₂ of 6.9 N (700 gf), an average minimum thickness t₂ of 4 μm, and a material thickness of the catheter of 90 μm. The flexural rigidity difference between the distal portion and the proximal portion was 313.81•10⁻³ N (32 gf), and the radio of flexural rigidity to the crushing strength of the proximal portion was 0.073.

The catheters obtained above are summarized in the table below.

[Table 1]

**Table 1**

| | | | | | | | | Flexural rigidity(gf) | | | Crushing strength(gf) | | | | |
|---|---|---|---|---|---|---|---|---|---|---|---|---|---|---|---|
| | OD (mm) | ID (mm) | Braid thickness (µm) | Catheter thickness (µm) | Calcd. minimum thickness (µm) | Measured minimum thickness (µm) | Resin (Shore D) | Distal | Proximal | Difference | Proximal | (Flex. rigid.) / (Crush. str.) | Anti-kinking str. (mm) | Tip shape restoration (%) | Back-up force (gf) |
| Example 1 | 1.43 | 1.27 | 25 | 80 | 20 | 8 | 68 | 8 | 17 | 9 | 530 | 0.032 | 3 | 80 | 4 |
| Example 2 | 1.43 | 1.27 | 25 | 80 | 20 | *8* | 57, 68, 78 | 8 | 12, 17, 21 | 13 | 450. 530, 600 | | 3 | 82 | 3 |
| Example 3 | 1.43 | 1.25 | 25 | 90 | 30 | 18 | 68 | 9 | 18 | 2 | 480 | 0.038 | 3 | 82 | 5 |
| Comp.Ex.1 | 1.40 | 1.27 | 25 | 65 | 5 | 4 | 68 | 9 | 17 | 11 | 450 | 0.038 | 15 | 65 | 2 |
| Comp.Ex.2 | 1.49 | 1.27 | 25 | 110 | 50 | 48 | 68 | 9 | 17 | 3 | 650 | 0.026 | not kinked | 85 | 6 |
| Comp.Ex.3 | 1.43 | 1.27 | 25 | 80 | 20 | 10 | 57 | 8 | 12 | 13 | 450 | 0.027 | not kinked | 92 | 1 |
| Comp.Ex.4 | 1.43 | 1.27 | 25 | 80 | 20 | 9 | 79 | 8 | 18 | 13 | 600 | 0.030 | 10 | 65 | 10 |
| Comp.Ex.5 | 1.41 | 0.97 | 25 | 220 | 160 | 120 | (urethane 60) | 10 | 18 | 13 | 935 | 0.019 | 3 | 80 | 12 |
| Comp.Ex.6 | 1.41 | 0.97 | 25 | 220 | 160 | 120 | (polyester 60) | 9 | 18 | 13 | 925 | 0.019 | 3 | 84 | 5 |
| Comp.Ex.7 | 1.73 | 1.50 | 30 | 115 | 45 | 39 | 78 | 8 | 45 | 37 | 880 | 0.051 | 12 | 84 | 8 |
| Comp.Ex.8 | 1.73 | 1.50 | 25 | 115 | 55 | 45 | 78 | 7 | 40 | 33 | 640 | 0.063 | 18 | - | - |
| Comp.Ex.9 | 2.06 | 1.80 | 35 | 130 | 50 | 28 | 78 | 15 | 70 | 55 | 1078 | 0.065 | 18 | 83 | 15 |
| Comp.Ex.10 | 1.43 | 1.17 | 25 | 130 | 70 | 55 | 68 | 8 | 18 | 13 | 580 | 0.031 | not kinked | 80 | 5 |
| Comp.Ex.11 | 1.43 | 1.20 | 25 | 115 | 55 | 43 | 68 | 9 | 19 | 10 | 550 | 0.035 | 3 | 80 | 5 |
| Comp.Ex.12 | 1.66 | 1.50 | 30 | 80 | 10 | 4 | 78 | 8 | 27 | 19 | 700 | 0.039 | 25 | 65 | 9 |
| Comp.Ex.13 | 2.06 | 1.88 | 35 | 90 | 10 | 5 | 68 | 8 | 40 | 32 | 550 | 0.073 | 30 | 65 | 10 |

The catheters obtained in Examples 1 to 3 and Comparative Examples 1 to 13 were evaluated by the test methods described below. The results are shown in Tables 2 and 3.
[Table 2]

**Table 2**

| | | | | Anti-kinking strength | Tip shape restoration performance | Back-up force | Animal experiment | Left coronary artery model |
|---|---|---|---|---|---|---|---|---|
| | Blood vessel trackability | Passage-through-stent performance | Device steerability | | | | Engageability (Shape retention) | Blood vessel trackability (Deformability) |
| Example 1 | ++ | +++ | ++ | ++ | ++ | ++ | ++ | ++ |
| Example 2 | +++ | +++ | ++ | ++ | +++ | + | ++ | +++ |
| Example 3 | ++ | +++ | + | ++ | +++ | ++ | +++ | ++ |
| Comp. Ex. 1 | ++ | + | ++ | - | + | - | - | ++ |
| Comp.Ex.2 | - | + | ++ | + | ++ | +++ | +++ | - |
| Comp.Ex.3 | - | + | ++ | +++ | +++ | - | - | - |
| Comp.Ex.4 | - | +++ | ++ | ++ | + | +++ | +++ | - |
| Comp.Ex.5 | - | - | - | ++ | ++ | +++ | +++ | - |
| Comp.Ex.6 | - | - | - | ++ | + | +++ | +++ | - |
| Comp.Ex.7 | - | - | +++ | ++ | ++ | +++ | +++ | - |
| Comp.Ex.8 | +++ | ++ | +++ | ++ | - | - | - | +++ |
| Comp.Ex.9 | - | - | +++ | +++ | +++ | +++ | +++ | - |
| Comp.Ex.10 | - | - | - | ++ | ++ | ++ | +++ | - |
| Comp.Ex.11 | + | + | - | ++ | ++ | ++ | +++ | + |
| Comp.Ex.12 | ++ | - | +++ | - | + | + | + | ++ |
| Comp.Ex.13 | + | - | +++ | - | + | + | + | + |

Table 2 shows respective property evaluation in the test results. Incidentally, "+++" in Table 2 means very good, "++" means good, "+" means poor, and "-" means very poor.

With respect to anti-kinking strength, the results of Comparative Examples 3 and 9 were the best, while the results of Comparative Examples 1, 12 and 13 were the poorest. The results of Examples 1 to 3 and Comparative Examples 4 to 8, 10 and 11 were medium.

With respect to flexural rigidity, Comparative Examples 1, 3 and 8 were low (flexible), while Comparative Examples 2 and 4 were high (hard). Examples 1 and 2 and Comparative Examples 5 and 6 were medium.

With respect to crushing strength, Comparative Examples 2, 5, 6, 7 and 9 were the highest, while Comparative Examples 1 and 3 were low. Examples 1 and 2 were medium.

With respect to tip shape restoration performance, Examples 2 and 3 and Comparative Examples 3 and 9 were high, while Example 1 and Comparative Examples 2, 5, 7, 10 and 11 were medium, and Comparative Examples 1, 4, 6, 8, 12 and 13 were low.

With respect to back-up force, Comparative Examples 2, 4 to 7 and 9 were high, while Comparative Examples 1, 3 and 8 were low. Examples 1, 3, 10 and 11 were medium.

Among these evaluations, the flexural rigidity represents deformability, and the back-up force represents shape retention performance.

From these evaluation results, it was verified that Examples 1 to 3 gave good balance of deformability and shape retention performance, which are contradictory properties.

### <Anti-kinking performance evaluation test (loop method)>

As shown in FIG. 16, a plate 501 having a thickness of 10 mm and provided with two holes 502 and 503 was prepared, wherein the holes 502 and 503 had a diameter of 2.8 mm and were so arranged that the center-to-center distance was 10 mm. Each catheter was passed through the two holes 502 and 503 so as to form a loop, then one end of the catheter was pulled to contract the loop, and, when a kink is generated in the loop portion, the distance L between the turn-back end of the loop and the plate 501 was measured. Incidentally, in this test, the measurement was conducted in 37°C warm water after the catheter was immersed in 37°C warm water for at least 30 minutes.

### <Flexural rigidity evaluation test>

The proximal end side of each catheter body was immersed in 37°C warm water for at least 30 minutes. Thereafter, as shown in FIG. 17, in 37°C warm water, the catheter was placed on a stainless steel jig provided with fulcrums having a height of 5 mm and arranged at an internal of 45 mm. A central portion of the catheter was pressed by use of a pressing tool (pressing member) having a radius of 5 mm at a rate of 5 mm/minutes, and the pressing force (rigidity) in this instance was measured.

### <Crushing strength evaluation test>

Each catheter body was immersed in 37°C warm water for at least 30 minutes. Thereafter, as shown in FIG. 18, in 37°C warm water, the catheter body was crushed by use of a pressing tool having a tip angle of 50° and a tip blade width of 0.2 mm, and the pressing force (strength) in this instance was measured.

### <Tip shape restoration performance evaluation test>

A distal portion of each of the catheters obtained in Examples and Comparative Examples above was processed into a Judkins left (JL) 4.0 shape, as shown in FIG. 19, and the angle α of the curved part was determined. Thereafter, the catheter was inserted into a sheath while being made rectilinear, and immediately pulled out. After 1 minute from the pulling-out, the angle β was measured. Restoration factor (%) was obtained according to the formula "(β/α) × 100."

### <Back-up force evaluation test>

A JL 4.0 shape portion was blanked, and was immersed in 37°C warm water for 30 minutes. Thereafter, a proximal portion of the guiding catheter was fixed in the warm water, and, as shown in FIG. 20, a thread attached to a distal portion of the catheter was pulled with such a tension that the curved part on the proximal side was opened to 90 degrees. The load in this instance was measured by an autograph AG-IS, produced by Shimadzu Corporation.

### <Animal Experiment 1>

A swine with a snaking present in the iliac artery was artificially made to suffer myocardial infarction by inserting a clot into the left coronary artery. The guiding catheter obtained in Example was inserted via a femoral region by an ordinary method using a 1.33 mm (4 Fr) introducer sheath (produced by Terumo Corporation). The distal end of the guiding catheter passed smoothly through the snaking portion, and was engaged with the left coronary artery. Via the distal end of the catheter, a guide wire (Runthrough NS Hypercoat, produced by Terumo Corporation) was inserted.

Subsequently, the guiding catheter obtained in each of Examples was inserted along the guide wire. The shape of the curved part of the guiding catheter was deformed according to the shapes of bent portions in the left coronary artery, and the guiding catheter could be advanced to a position immediately on the proximal side of a lesion part, without any large resistance or catching. A balloon catheter (produced by Terumo Corporation) was inserted via the distal opening of the guiding catheter, and the lesion part was dilated.

It was confirmed that the lesion part was dilated appropriately and the stenosis was eliminated. After the balloon catheter was pulled out, a drug releasing stent was inserted, was expanded in the lesion part, and was left indwelling there.

The steerability of the device was good, and the distal end was rotated in response to application of torque at the hub, without generation of torsion or kinking.

In addition, during operation, not any ischemia or exfoliation of blood vessel wall was observed. After the dilating work was over, both the treatment catheter such as balloon catheter and the guiding catheter used for the work could be pulled out easily.

The test was similarly conducted for each of the catheters obtained respectively in Comparative Examples. The catheter of Comparative Example 1 showed a sharp bending at the curved part in the stage of insertion into the blood vessel. In the case of the catheter of Comparative Example 2, the curved part could be inserted via the femoral artery, the curved part was stuck on the blood vessel wall, to make it difficult to advance the catheter into the coronary artery. When it was tried to insert the catheter further, the curved part would not deform according to the shape of the blood vessel. In the case of the catheter of Comparative Example 3, the curved part could be inserted smoothly to the coronary artery, but the curved part was easily disengaged upon engagement with the coronary artery orifice, so that it was impossible to insert the treatment catheter. In the case of the catheter of Comparative Example 4, the curved part could smoothly be engaged with the coronary artery orifice, but in the coronary artery it would not be deformed according to the shape of the blood vessel, so that it was impossible to advance the curved part to the lesion part (see FIG. 15).

### <Animal Experiment 2>

The same method as in Animal Experiment 1 was used, except that a clot was inserted into a right coronary artery.

The catheter obtained in each Example processed into a JR 3.5 shape was engaged with the right coronary artery. A drug releasing stent was inserted at a position on the proximal side relative to the clot, was expanded in a lesion part, and was left indwelling there.

Subsequently, a guide wire (produced by Terumo Corporation) was inserted into the coronary artery, whereon the guide wire was caught on the stent. In response to this situation, the guide wire was returned into the catheter, and the catheter was inserted into the stent. The catheter reached a position just on the proximal side of the lesion part, without being caught on the stent.

A balloon catheter (produced by Terumo Corporation) was inserted via the distal opening part (distal opening 41) of the catheter, and operated to dilate the lesion part.

It was confirmed that the lesion part was dilated appropriately and the stenosis was eliminated. After the balloon catheter was pulled out, a drug releasing stent was inserted, was expanded in the lesion part, and was left indwelling there.

The steerability of the device was good, and the distal end was rotated in response to application of torque at the hub, without generation of torsion or kinking.

In addition, during operation, not any ischemia or exfoliation of blood vessel wall was observed. After the dilating work was over, both the treatment catheter such as balloon catheter and the catheter could be pulled out easily, without being caught on the blood vessel or the stent initially put indwelling.

The test was similarly conducted for each of the catheters obtained respectively in Comparative Examples. The catheter of Comparative Example 1 showed a sharp bending at the curved part in the stage of insertion into the blood vessel. In the case of the catheter of Comparative Example 2, the curved part could be inserted via the femoral artery, the curved part was stuck on the blood vessel wall, to make it difficult to advance the catheter into the coronary artery. When it was tried to insert the catheter further, the curved part would not be deformed according to the shape of the blood vessel. In the case of the catheter of Comparative Example 3, the curved part could be inserted smoothly to the coronary artery, but the curved part was easily disengaged upon engagement with the coronary artery orifice, so that it was impossible to insert the device. In the case of the catheter of Comparative Example 4, the curved part could smoothly be engaged with the coronary artery orifice, but in the coronary artery it would not be deformed according to the shape of the blood vessel, so that it was impossible to advance the curved part to the lesion part.

In the cases of the catheters of Examples, such a series of operations as above-mentioned could be performed assuredly (see Table 2). Particularly, the catheter of Comparative Example 5 could be engaged with the swine coronary artery, but it would not be deformed in the coronary artery, so that it could not be advanced to the periphery of the coronary artery. The catheter of Comparative Example 6 could not be engaged with the swine coronary artery orifice (see Table 2).

### <Test using left coronary artery model>

As shown in FIG. 21, a test was conducted wherein the guiding catheter of each Example was inserted into a model imitating a left coronary artery, and it was evaluated whether or not the catheter could be inserted along the blood vessel without any unwilling deflection or curving.

The catheter was advanced via a femoral artery to the entrance to left coronary artery (the branched portion). A distal portion of the catheter was engaged with the entrance to left coronary artery (the branched portion), and the distal portion was inserted toward the distal side inside the left coronary artery. As the catheter was advanced toward the distal side, the distal portion of the catheter was deformed according to the shapes of the curved portions of the left coronary artery (a curved portion of R15 (radius of curvature: 15 mm), a curved portion of R10, a curved portion of R7.5, and a curved portion of R4). The catheter could be advanced, without any large resistance or catching. In the case of the catheter of each Example, the distal portion passed through the R4 curved portion, to reach a rectilinear portion on the distal side of the R4 curved portion. The results are shown in "Blood vessel trackability (Deformability)" in Table 2.

### <Clinical Trial 1>

First, following a common procedure, the distal end of a catheter is advanced to a starting portion of an aorta. Specifically, for example, according to the Seldinger technique, a catheter introducer is made to puncture a femoral artery, and a catheter in the condition in which a guide wire is inserted in a predetermined position is inserted into the sheath of the catheter introducer. While letting the guide wire precede, the distal end of the catheter is introduced via a distal hole of the sheath into the femoral artery. Next, the catheter is advanced gradually, to be inserted into the femoral artery. In this case, in order to pass the distal end of the catheter through bent portions of the blood vessel or in order to select a branch of the blood vessel, operations including an appropriate combination of letting-in and letting-out of the guide wire and forward movement, backward movement and rotation of the catheter are carried out.

Next, upon the advancement of the distal end of the catheter to the starting portion of the aorta, the guide wire is pulled out, and such an operation as to let the distal end of the catheter reach a left coronary artery sinus is conducted.

Subsequently, in order to check the position of the left coronary artery orifice, test radioscopic imaging is performed. After the position of the left coronary artery orifice can be confirmed, the catheter is moved to slowly insert the distal end thereof into the left coronary artery.

Next, while letting the patient take a deep breath, the catheter 1 is advanced a little to insert the distal end thereof into a left main trunk part.

A guide wire (diameter: "0014, produced by Terumo Corporation) is inserted into the coronary artery via the distal end of the catheter, and is caused to reach a lesion part (AHA classification: #6).

Subsequent to such operations as above-mentioned, the catheter 1 was inserted along the guide wire. The shape of the distal portion of the catheter 1 was deformed according to the shapes of bent portions in the left coronary artery, and the catheter 1 was advanced to a position just on the proximal side of the lesion part, without any large resistance or catching. A balloon catheter (produced by Terumo Corporation) was inserted via the distal opening part of the catheter 1, and operated to dilate the lesion part.

It was confirmed that the lesion part was dilated appropriately and the stenosis was eliminated. After the balloon catheter was pulled out, a bare metal stent was further inserted, was expanded in the lesion part, and was left indwelling there.

The steerability of the device was good, and the distal portion was rotated in response to application of torque at the hub, without generation of torsion or kinking.

In addition, during operation, not any ischemia or exfoliation of blood vessel wall was observed. After the dilating work was over, both the treatment catheter such as balloon catheter and the catheter 1 could be pulled out easily.

### <Clinical Trial 2>

The same method as in Clinical Trial 1 was used, except that a catheter 1 processed into a JR 3.5 shape was engaged with a right coronary artery. Next, the catheter 1 is advanced a little to insert its distal opening 41 into the right coronary artery.

A guide wire (diameter: "0014, produced by Terumo Corporation) is inserted into the coronary artery via the distal end of the catheter 1, and is caused to reach a lesion part (AHA classification: #3).

Subsequent to such operations as above-mentioned, the catheter 1 was inserted along the guide wire. The shape of the distal portion of the catheter 1 was deformed according to the shapes of bent portions in the right coronary artery, and the catheter 1 was advanced to a position just on the proximal side of the lesion part, without any large resistance or catching. A balloon catheter (produced by Terumo Corporation) was inserted via the distal opening part of the catheter 1, and operated to dilate the lesion part.

It was confirmed that the lesion part was dilated appropriately, and the stenosis was eliminated. After the balloon catheter was pulled out, a bare metal stent (produced by Terumo Corporation) was further inserted, was expanded in the lesion part, and was left indwelling there.

It was confirmed that the stenosis was removed and the bloodstream was recovered, after the indwelling of the stent.

### <Clinical Trial 3>

The same method as in Clinical Trial 1 was used, except that a 1.33 mm (4 Fr) introducer sheath (produced by Terumo Corporation) is made to puncture a radial artery instead of the femoral artery and that a catheter 1 processed into a JR 3.5 shape is inserted into the sheath of the catheter introducer and engaged with a right coronary artery.

The catheter 1 is advanced a little to insert its distal opening 41 into the right coronary artery.

A guide wire (diameter: "0014, produced by Terumo Corporation) is inserted into the coronary artery via the distal end of the catheter 1, and made to reach a lesion part (AHA classification: #3).

Subsequent to such operations as above-mentioned, the catheter 1 was inserted along the guide wire. The shape of the distal portion of the catheter 1 was deformed according to the shapes of bent portions in the right coronary artery, and the catheter 1 was advanced to a position just on the proximal side of the lesion part, without any large resistance or catching. A bare metal stent was inserted via the distal opening part of the catheter 1, was expanded in the lesion part, and was left indwelling there.

It was confirmed that the lesion part was dilated appropriately and the stenosis was eliminated. After the balloon catheter was pulled out, a bare metal stent was further inserted, was expanded in the lesion part, and was left indwelling there.

It was confirmed that the stenosis was removed and the bloodstream was recovered, after the indwelling of the stent.

### <Clinical Trial 4>

The same method as in Clinical Trial 3 was used, except that a catheter 1 having a JL 4.0 shape is engaged with a left coronary artery. In view of that a bare metal stent (produced by Terumo Corporation) had already been left indwelling (AHA classification: #6), a guide wire (diameter: "0014, produced by Terumo Corporation) was returned into the catheter 1, and the catheter 1 was inserted into the left coronary artery. Upon this operation, the catheter 1 was deformed according to the shapes of bent portions in the left coronary artery; thus, the catheter 1 was advanced to a position just on the proximal side of the lesion part (AHA classification: #8) through the inside of the already indwelling bare metal stent, without any large resistance or catching.

Via the distal opening part of the catheter 1, the guide wire and a balloon catheter (produced by Terumo Corporation) were inserted into the blood vessel, and the lesion part was dilated.

It was confirmed that the lesion part was dilated appropriately and the stenosis was eliminated. After the balloon catheter was pulled out, a bare metal stent was further inserted, was expanded in the lesion part, and was left indwelling there. It was confirmed that the stenosis was removed and the bloodstream was recovered, after the indwelling of the stent.

### <Clinical Trial 5>

The same method as in Clinical Trial 2 was used, except that a catheter 1 having a JR 3.5 shape and a size of Example 2 is engaged with a right coronary artery. Next, the catheter 1 is advanced a little to insert its distal opening 41 into the right coronary artery.

A guide wire (diameter: "0014, produced by Terumo Corporation) is inserted into the coronary artery via the distal end of the catheter 1, and was caused to reach a lesion part (AHA classification: #2).

Subsequent to such operations as above-mentioned, the catheter 1 was inserted along the guide wire. The shape of a distal portion of the catheter 1 was deformed according to the shapes of bent portions in the right coronary artery, and the catheter 1 was advanced to a position just on the proximal side of the lesion part, without any large resistance or catching. A balloon catheter (produced by Terumo Corporation) was inserted via the distal opening part of the catheter 1, and operated to dilate the lesion part.

It was confirmed that the lesion part was dilated appropriately and the stenosis was eliminated. After the balloon catheter was pulled out, a bare metal stent (produced by Terumo Corporation) was further inserted, was expanded in the lesion part, and was left indwelling there.

It was confirmed that the stenosis was removed and the bloodstream was recovered, after the indwelling of the stent.

### <Passage-through-stent performance test>

[Table 3]

**Table 3**

| | Test A | | Test B |
|---|---|---|---|
| | Tip deformation | Catching | Resistance value *N* (gf) |
| Example 1 | absent | absent | 1.25 (127) |
| Example 2 | absent | absent | 1.13 (115) |
| Example 3 | absent | absent | 1.20 (122) |
| Comparative Example 7 | present | present | not measurable |
| Comparative Example 8 | absent | absent | 1.99 (203) |

Table 3 shows the results of a passage performance test for testing the passage of a catheter through the inside of a stent set indwelling in a model imitating a blood vessel (coronary artery) (for example, the condition shown in FIG. 14).

In Test A in Table 3, first, a stent (TUNAMI GOLD (4.0), produced by Terumo Corporation) is expanded in an acrylic resin tube having an inside diameter of 3 mm, and a guide wire (RunthrouNS Floppy, produced by Terumo Corporation) is inserted into and passed through the stent. Next, each of the catheters (Judkins left (JL) 4.0) obtained in Examples 1 to 3 and Comparative Examples 7 and 8 is reciprocatingly slid twenty times along the guide wire. The state of the soft tip of each catheter during such an operation was observed. In the cases of the catheters of Examples 1 to 3 and Comparative Example 8, the catheter could pass through the stent without any soft tip deformation or catching. On the other hand, in the case of the catheter of Comparative Example 7, the catheter could not pass through the stent due to soft tip deformation or catching. Comparative Examples 1 to 6 and 9 to 13 also gave results similar to that of Comparative Example 7.

In Test B in Table 3, in the condition where the soft tip of a catheter is forcibly hooked on a stent strand, the catheter is pushed forward by use of a force gage. The resistance value (measurement) indicated by the force gage in this instance was recorded. In the cases of the catheters of Examples 1 to 3, the resistance value was within an allowable range (0.78 to 1.47 N) (80 to 150 gf) such that the catheter can smoothly pass through the inside of the stent. On the other hand, in the cases of the catheters of Comparative Examples 7 and 8, the resistance value was outside the allowable range or could not be measured. Comparative Examples 1 to 6 and 9 to 13 also gave results similar to that of Comparative Example 7 or 8.

From the results of Tests A and B as above, it is seen that the catheters obtained in Examples 1 to 3 can pass through the stent smoothly (swiftly).

### Industrial Applicability

The catheter according to the present invention is a catheter to be used in the state of being inserted in a blood vessel, and includes a catheter body having an inner layer, an outer layer and a reinforcing material layer located between them and a soft tip which is provided on the distal side of the catheter body and more flexible than the catheter body. The catheter body has, at its distal portion, a curved part which has a curved shape and which is engageable with a branched portion of the blood vessel due to the curved shape. The curved part is so configured that the total thickness of the inner layer, the outer layer and the reinforcing material layer is 60 to 100 µm and the minimum thickness of the outer layer is 8 to 30 µm. This ensures that when the curved part is pushed forward to the distal side relative to the branched portion, the curved part is deformed according to the shape of the distal side portion of the blood vessel. Therefore, due to the presence of the outer layer which is comparatively hard but is thin, the curved part retains a shape necessary for engagement with the branched portion (entrance) of the target blood vessel (has a shape retention performance). Furthermore, when the curved part is inserted to the distal side of the branched portion of the blood vessel (for example, into a coronary artery), the curved part is deformed according to the shape of the blood vessel (has a property to be deformable without crushing). This ensures that the catheter can be assuredly inserted to a target site in a blood vessel through a simple operation and in a safe and assured manner. Accordingly, the catheter of the present invention has industrial applicability.

## Claims

1. A guiding catheter (1) having an outside diameter of 1.4 to 1.6 mm which is to be inserted into a blood vessel and which is capable of being used alone for the purpose of guiding, i.e. being used singly as a guiding catheter and not used together with other guiding catheter or support tube for the purpose of guiding, the catheter comprising:
a catheter body (3) which has an inner layer (34), an outer layer (35) and a reinforcing material layers (36) located therebetween; and
a soft tip (4) which is provided on the distal side of the catheter body and more flexible than the catheter body;
wherein the catheter body (3) has, at its distal portion, a curved part (33) which has a curved shape and is engageable with a branched portion of the blood vessel due to the curved shape, and
the flexural rigidity of the distal-side portion of the catheter body is smaller than the flexural rigidity of the proximal-side portion of the catheter body by 49.03 to 196.13 · 10⁻³ N (5 to 20 gf), and
the curved part (33) is so configured that the average total thickness of the inner layer (34), the outer layer (35) and the reinforcing material layer (36) is 60 to 100 µm and the average minimum thickness of the outer layer is 8 to 30 µm, whereby, when the curved part (33) is pushed from the branched portion toward a distal side, the curved part (33) can be deformed according to the shape of a portion on the distal side of the blood vessel,
wherein the guiding catheter is designed such that if it is carried alone to the coronary artery orifice of the coronary artery having a stenosis, then a distal portion of the guiding catheter is inserted into the coronary artery orifice, and thereby the guiding catheter is immobilized in that position, prior to insertion of a treatment catheter, wherein the portion on the distal side of the blood vessel is a coronary artery, and
the catheter (1) is configured and adapted to be passed through a lumen of a stent having an inside diameter of 2.2 to 3.0 mm which is disposed inside the coronary artery.

2. The catheter (1) according to claim 1, wherein the minimum thickness of the outer layer (35) is the distance from an outermost periphery of the reinforcing material layer (36) to an outside surface of the outer layer (35).

3. The catheter (1) according to claim 1, wherein let the average minimum thickness of the outer layer (35) be t and let the outside diameter ø of the catheter body be d, then d/t is in the range of from 50 to 250.

4. The catheter (1) according to claim 1, wherein let the flexural rigidity of a proximal portion (31) of the catheter body (3) be σ1 [gf] and let the crushing strength of the proximal portion (31) be σ2 [gf], then the condition of σ1/σ2 ≤ 0.04 is satisfied.

5. The catheter (1) according to claim 1, wherein the outer layer (35) is formed from different two or more materials along a longitudinal direction of the catheter body (3)

6. The catheter (1) according to claim 1, wherein the outside diameter of the catheter body (3) is 1.4 to 1.5 mm.

7. The catheter (1) according to claim 1, wherein the inside diameter of the catheter body (3) is 1.2 to 1.5 mm.

8. The catheter (1) according to claim 1, wherein the outer layer (35) is provided in its surface with a multiplicity of recesses.

9. The catheter (1) according to claim 8, wherein the average of maximum depths of the recesses (356) is not more than 10 µm.

10. The catheter (1) according to claim 1, wherein the curved part is a part having a shape which is curved only toward the same direction.

11. The catheter (1) according to claim 1, wherein the curved part (33) is so configured that a portion having a curvature increasing along the distal direction and a portion having a curvature decreasing along the distal direction are alternately arranged at least once along the longitudinal direction of the catheter body (3).

12. The catheter (1) according to claim 1, wherein at least part of the inner layer (34) is formed from a low-friction material.

13. The catheter (1) according to claim 1, wherein the soft tip (4) is formed from the same material as that of the outer layer (35) and is formed as one body with the outer layer.

14. The catheter (1) according to claim 1, being a guiding catheter for introducing a balloon catheter, a stent transport catheter or a vasodilation catheter.

15. The catheter (1) according to claim 1, wherein the average total thickness of the inner layer (34), the outer layer (35) and the reinforcing material layer (36) is 75 to 85 µm.

16. The catheter (1) according to claim 1, wherein at least part of an outside surface of the outer layer (35) at a distal portion of the catheter (1) is provided with a coating which exhibits swellability upon contact with a liquid.

## Patentansprüche

1. Führungskatheter (1) mit einem Außendurchmesser von 1,4 bis 1,6 mm, welcher in ein Blutgefäß einzuführen ist und welcher dazu geeignet ist, zum Zweck des Führens allein verwendet zu werden, d. h. als Führungskatheter allein und nicht zusammen mit einem anderen Führungskatheter oder Trägerrohr zum Zweck der Führung zu verwenden ist, wobei der Katheter umfasst:
einen Katheterkörper (3), welcher eine innere Schicht (34), eine äußere Schicht (35) und eine dazwischen angeordnete Verstärkungsmaterial-Schicht (36) aufweist; und
eine weiche Spitze (4), welche auf der distalen Seite des Katheterkörpers vorgesehen ist und flexibler ist als der Katheterkörper;
wobei der Katheterkörper (3) an seinem distalen Abschnitt ein gebogenes Teil (33) aufweist, welches eine gebogene Form aufweist und aufgrund der gebogenen Form mit einem Verzweigungsabschnitt des Blutgefäßes in Eingriff gebracht werden kann, und wobei
die Biegesteifigkeit des distal-seitigen Abschnitts des Katheterkörpers um 49,03 bis 196,13 • 10⁻³N (5 bis 20 gf) geringer ist als die Biegesteifigkeit des proximal-seitigen Abschnitts des Katheterkörpers, und wobei
das gebogene Teil (33) so angepasst ist, dass die durchschnittliche Gesamtdicke der inneren Schicht (34), der äußeren Schicht (35) und der Verstärkungsmaterial-Schicht (36) 60 bis 100 µm und die durchschnittliche minimale Dicke der äußeren Schicht 8 bis 30 µm beträgt, wodurch, wenn das gebogene Teil (33) vom Verzweigungsabschnitt hin zu einer distalen Seite gedrückt wird, das gebogene Teil (33) gemäß der Form eines Abschnitts auf der distalen Seite des Blutgefäßes verformt werden kann,
wobei der Führungskatheter so ausgestaltet ist, dass, wenn er allein zur Koronararterienöffnung der eine Stenose aufweisenden Koronararterie geführt wird, dann ein distaler Abschnitt des Führungskatheters in die Koronararterienöffnung eingeschoben wird und dadurch der Führungskatheter in dieser Position vor dem Einschub eines Behandlungskatheters immobilisiert wird, wobei der Abschnitt auf der distalen Seite des Blutgefäßes eine Koronararterie ist, und wobei
der Katheter (1) so konfiguriert und angepasst ist, um durch ein Lumen eines Stents mit einem Innendurchmesser von 2,2 bis 3,0 mm geschoben werden zu können, welcher innerhalb der Koronararterie angeordnet ist.

2. Katheter (1) gemäß Anspruch 1, wobei die minimale Dicke der äußeren Schicht (35) der Abstand von einer äußersten Seite der Verstärkungsmaterial-Schicht (36) zu einer äußeren Oberfläche der äußeren Schicht (35) ist.

3. Katheter (1) gemäß Anspruch 1, wobei wenn, angenommen dass die durchschnittliche minimale Dicke der äußeren Schicht (35) t ist und angenommen dass der Außendurchmesser ø des Katheterkörpers d ist, d/t im Bereich von 50 bis 250 liegt.

4. Katheter (1) gemäß Anspruch 1, wobei wenn, angenommen dass die Biegesteifigkeit eines proximalen Abschnitts (31) des Katheterkörpers (3) σ1 [gf] ist und angenommen dass die Stoßhärte des proximalen Abschnitts (31) σ2 [gf] ist, die Bedingung σ1/σ2 ≤ 0,04 erfüllt ist.

5. Katheter (1) gemäß Anspruch 1, wobei die äußere Schicht (35) aus verschiedenen zwei oder mehr Materialien entlang einer Längsrichtung des Katheterkörpers (3) gebildet ist.

6. Katheter (1) gemäß Anspruch 1, wobei der Außendurchmesser des Katheterkörpers (3) 1,4 bis 1,5 mm beträgt.

7. Katheter (1) gemäß Anspruch 1, wobei der Innendurchmesser des Katheterkörpers (3) 1,2 bis 1,5 mm beträgt.

8. Katheter (1) gemäß Anspruch 1, wobei die äußere Schicht (35) auf ihrer Oberfläche mit einer Vielzahl von Aussparungen versehen ist.

9. Katheter (1) gemäß Anspruch 8, wobei der Durschnitt von maximalen Tiefen der Aussparungen (356) nicht mehr als 10 µm beträgt.

10. Katheter (1) gemäß Anspruch 1, wobei das gebogene Teil ein Teil ist, welcher eine Form besitzt, welche lediglich hin zur selben Richtung gebogen ist.

11. Katheter (1) gemäß Anspruch 1, wobei der gebogene Teil (33) so ausgestaltet ist, dass ein Abschnitt, welcher eine sich entlang einer distalen Richtung verstärkende Biegung besitzt, und ein Abschnitt, welcher eine sich entlang der distalen Richtung abschwächende Biegung besitzt, mindestens einmal entlang der Längsrichtung des Katheterkörpers (3) abwechselnd angeordnet sind.

12. Katheter (1) gemäß Anspruch 1, wobei mindestens ein Teil der inneren Schicht (34) aus einem Material mit niedriger Reibung ausgebildet ist.

13. Katheter (1) gemäß Anspruch 1, wobei die weiche Spitze (4) aus demselben Material ausgebildet ist wie das der äußeren Schicht (35) und als ein Körper mit der äußeren Schicht ausgebildet ist.

14. Katheter (1) gemäß Anspruch 1, welches ein Führungskatheter zum Einführen eines Ballonkatheters, eines Transportkatheters für einen Stent oder eines Vasodilatationskatheters ist.

15. Katheter (1) gemäß Anspruch 1, wobei die durchschnittliche Gesamtdicke der inneren Schicht (34), der äußeren Schicht (35) und der Verstärkungsmaterial-Schicht (36) 75 bis 85 µm beträgt.

16. Katheter (1) gemäß Anspruch 1, wobei mindestens ein Teil der Außenoberfläche der äußeren Schicht (35) an einem distalen Abschnitt des Katheters (1) mit einer Beschichtung versehen ist, welche Schwellfähigkeit bei Kontakt mit einer Flüssigkeit aufweist.

## Revendications

1. Cathéter-guide (1) ayant un diamètre extérieur de 1,4 à 1,6 mm qui doit être inséré dans un vaisseau sanguin et qui est apte à être utilisé seul dans le but de guider, c'est-à-dire être utilisé séparément comme cathéter-guide et non utilisé avec un autre cathéter-guide ou tube de support dans le but de guider, le cathéter comprenant :
un corps de cathéter (3) qui possède une couche intérieure (34), une couche extérieure (35) et une couche de matériau de renforcement (36) située entre elles ; et
une pointe molle (4) qui est disposée sur le côté distal du corps de cathéter et plus souple que le corps de cathéter ;
dans lequel le corps de cathéter (3) comporte, au niveau de sa portion distale, une partie courbe (33) qui présente une forme incurvée et peut s'engager dans une portion ramifiée du vaisseau sanguin en raison de la forme incurvée, et
la rigidité en flexion de la portion côté distal du corps de cathéter est plus faible que la rigidité en flexion de la portion côté proximal du corps de cathéter de 49,03 à 196,13·10⁻³ N (5 à 20 gf), et
la partie courbe (33) est configurée de telle sorte que l'épaisseur totale moyenne de la couche intérieure (34), de la couche extérieure (35) et de la couche de matériau de renforcement (36) est de 60 à 100 µm et l'épaisseur minimale moyenne de la couche extérieure est de 8 à 30 µm, moyennant quoi, lorsque la partie courbe (33) est poussée depuis la portion ramifiée vers un côté distal, la partie courbe (33) peut être déformée en fonction de la forme d'une portion sur le côté distal du vaisseau sanguin,
dans lequel le cathéter-guide est conçu de telle sorte que, s'il est transporté seul jusqu'à l'orifice d'artère coronaire de l'artère coronaire ayant une sténose, alors une partie distale du cathéter-guide est insérée dans l'orifice d'artère coronaire, et de ce fait le cathéter-guide est immobilisé dans cette position, avant insertion d'un cathéter de traitement,
dans lequel la portion sur le côté distal du vaisseau sanguin est une artère coronaire, et
le cathéter (1) est configuré et adapté pour être passé à travers une lumière d'un stent ayant un diamètre intérieur de 2,2 à 3,0 mm qui est disposé à l'intérieur de l'artère coronaire.

2. Cathéter-guide (1) selon la revendication 1, dans lequel l'épaisseur minimale de la couche extérieure (35) est la distance depuis une périphérie la plus extérieure de la couche de matériau de renforcement (36) jusqu'à une surface extérieure de la couche extérieure (35).

3. Cathéter (1) selon la revendication 1, dans lequel, si l'épaisseur minimale moyenne de la couche extérieure (35) est t et si le diamètre extérieur 0 du corps de cathéter est d, alors d/t se trouve dans la plage de 50 à 250.

4. Cathéter (1) selon la revendication 1, dans lequel, si la rigidité en flexion d'une portion proximale (31) du corps de cathéter (3) est σ1 [gf] et si la résistance à l'écrasement de la portion proximale 31 est σ2 [gf], alors la condition de σ1/σ2 ≤ 0,04 est satisfaite.

5. Cathéter (1) selon la revendication 1, dans lequel la couche extérieure (35) est composée de deux matériaux différents ou plus, le long d'une direction longitudinale du corps de cathéter (3).

6. Cathéter (1) selon la revendication 1, dans lequel le diamètre extérieur du corps de cathéter (3) est de 1,4 à 1,5 mm.

7. Cathéter (1) selon la revendication 1, dans lequel le diamètre intérieur du corps de cathéter (3) est de 1,2 à 1,5 mm.

8. Cathéter (1) selon la revendication 1, dans lequel la couche extérieure (35) est pourvue, sur sa surface, d'une multiplicité d'évidements.

9. Cathéter (1) selon la revendication 8, dans lequel la moyenne des profondeurs maximales des évidements (356) n'excède pas 10 µm.

10. Cathéter (1) selon la revendication 1, dans lequel la partie courbe est une partie présentant une forme qui est incurvée seulement vers la même direction.

11. Cathéter (1) selon la revendication 1, dans lequel la partie courbe (33) est configurée de telle sorte qu'une portion présentant une courbure augmentant le long de la direction distale et une portion présentant une courbure diminuant le long de la direction distale sont agencées en alternance au moins une fois le long de la direction longitudinale du corps de cathéter (3).

12. Cathéter (1) selon la revendication 1, dans lequel au moins une partie de la couche intérieure (34) est composée d'un matériau à faible friction.

13. Cathéter (1) selon la revendication 1, dans lequel la pointe molle (4) est composée du même matériau que celui de la couche extérieure (35) et est formée d'un seul tenant avec la couche extérieure.

14. Cathéter (1) selon la revendication 1, qui est un cathéter-guide pour introduire un cathéter à ballonnet, un cathéter d'acheminement de stent ou un cathéter à vasodilatation.

15. Cathéter (1) selon la revendication 1, dans lequel l'épaisseur totale moyenne de la couche intérieure (34), de la couche extérieure (35) et la couche de matériau de renforcement (36) est de 75 à 85 µm.

16. Cathéter (1) selon la revendication 1, dans lequel au moins une partie d'une surface extérieure de la couche extérieure (35) au niveau d'une portion distale du cathéter (1) est pourvue d'un revêtement qui présente une aptitude au gonflement lors d'un contact avec un liquide.
